# EUROPEAN PATENT APPLICATION

(11) **EP 4 544 987 A1**
(43) Date of publication of application: **30.04.2025**
(21) Application number: 23830868.8
(22) Date of filing: 17.05.2023
(51) Int. Cl.: A61B 3/113, G01B 11/26

(54) **LINE-OF-SIGHT DETECTION DEVICE**

(30) Priority: 27.06.2022 JP 2022102503
(71) Applicant: Sony Group Corporation, Tokyo 108-0075 (JP)
(72) Inventor: MAMISHIN, Yuki, Tokyo 108-0075 (JP)
(74) Representative: MFG Patentanwälte Meyer-Wildhagen Meggle-Freund Gerhard PartG mbB
(86) International application number: PCT/JP2023/018364
(87) International publication number: WO 2024/004416

(57) **Abstract**

Provided is a gaze detection device that can suppress reduction in detection accuracy regardless of the imaging method of an imaging system.

The gaze detection device according to the present technique includes a patterned-light illumination system that irradiates an eyeball with patterned light, an imaging system that captures an image of the eyeball irradiated with the patterned light, and a gaze detection system that detects a gaze as the direction of the eyeball on the basis of the captured image of the imaging system. The patterned light has a shape along the reference pattern having at least three lines not parallel to one another, the reference pattern having an intersection point where at least two lines of the at least three lines cross each other. According to the gaze detection device of the present technique, the gaze detection device can be provided to suppress reduction in detection accuracy regardless of the imaging method of the imaging system.

## Description

### [Technical Field]

A technique according to the present disclosure (hereinafter also referred to as the "present technique") relates to a gaze detection device.

### [Background Art]

A conventionally known gaze detection device detects gaze, i,e., the direction of an eyeball, by irradiating the eyeball with patterned light and imaging the eyeball with an imaging system.

Conventional gaze detection devices include a device that irradiates an eyeball with patterned light shaped like a rectangular frame (for example, see PTL 1) and a device that irradiates an eyeball with patterned light shaped like two line segments crossing at one point (for example, see PTL 2).

### [Citation List]

### [Patent Literatures]

[PTL 1] JP 2010-250789 A
[PTL 2] JP 2014-188322 A

### [Summary]

### [Technical Problem]

However, the conventional gaze detection devices may reduce detection accuracy depending upon the imaging method (e.g., event-based vision method) of an imaging system.

Hence, an object of the present technique is to provide a gaze detection device that can suppress reduction in detection accuracy regardless of the imaging method of an imaging system.

### [Solution to Problem]

The present technique provides a gaze detection device including: a patterned-light illumination system that irradiates an eyeball with patterned light;
an imaging system that captures an image of the eyeball irradiated with the patterned light; and
a gaze detection system that detects a gaze as the direction of the eyeball on the basis of the captured image of the imaging system, wherein
the patterned light has a shape along a reference pattern having at least three lines not parallel to one another, the reference pattern having an intersection point where at least two lines of the at least three lines cross each other.

Each of the at least three lines may cross at least one of the other lines.

Each of the at least three lines may cross at least two of the other lines.

The reference pattern may have the at least three intersection points not located on the same straight line.

The at least three lines may cross one another at the same intersection point.

The at least three lines may form consecutive acute angles around the intersection point.

The at least three lines may extend radially from the intersection point.

The reference pattern may have multiple patterns, each including the at least two lines crossing each other.

The multiple patterns may include the at least two patterns that are identical in shape.

The multiple patterns may include the at least two patterns of different shapes.

The patterned-light illumination system may irradiate the eyeball with portion of the patterned light, the portion corresponding to the pattern where the intersection point is detectable among the multiple patterns.

The gaze detection system may include a corneal-reflection-image detection unit that detects from the captured image a feature point of a corneal reflection image of the patterned light.

The corneal-reflection-image detection unit may associate the intersection point and the feature point on the basis of information indicating an angle and/or a direction near the feature point of the corneal reflection image in the captured image.

The corneal-reflection-image detection unit may extract a pixel that has been increased in brightness in the captured image, as a pixel constituting the feature point.

The gaze detection system may include a movement information estimation unit that estimates at least one of the amount of movement, the direction of movement, and the velocity of movement of the eyeball on the basis of a detection result in the corneal-reflection-image detection unit.

The movement information estimation unit may estimate the position of the feature point that corresponds to the intersection point and that has not been detected by the corneal-reflection-image detection unit, from the feature point that corresponds to the intersection point and that has been detected by the corneal-reflection-image detection unit, and then extract from the estimation result the direction of movement of the eyeball.

The gaze detection system may detect the gaze on the basis of at least a captured image of the imaging system and the detection result in the corneal-reflection-image detection unit.

The patterned-light illumination system may include a light source that emits the patterned light.

The patterned-light illumination system may include a light source and an optical element that shapes light from the light source to generate the patterned light.

The imaging system may include an event sensor.

### [Brief Description of Drawings]

[Fig. 1]

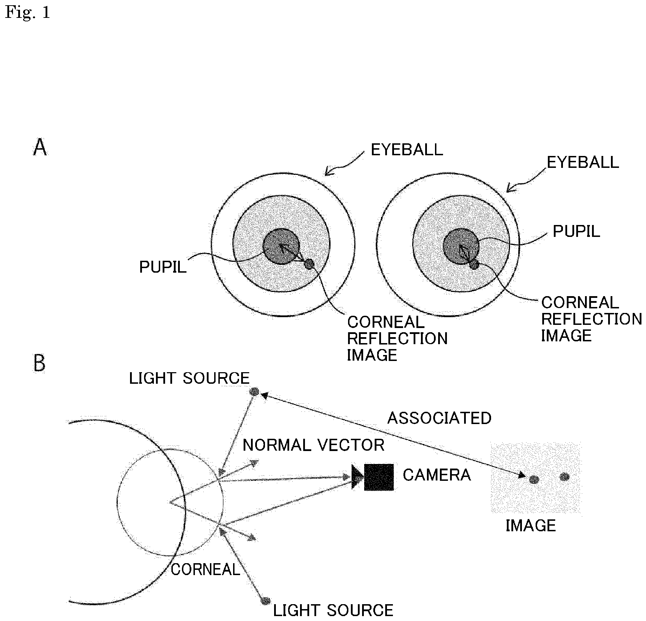

   Figs. 1A and 1B are explanatory drawings of a conventional eye tracking method (1 and 2).
[Fig. 2]

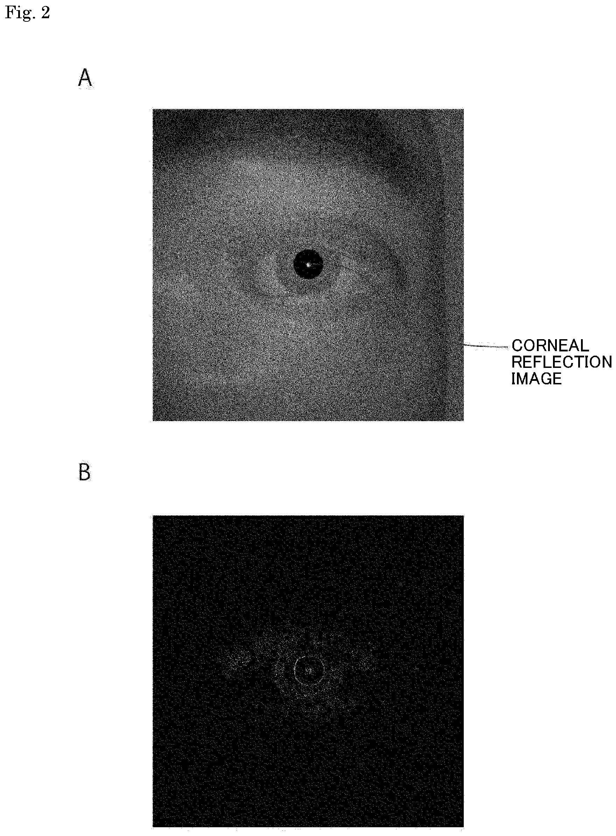

   Figs. 2A and 2B are explanatory drawings illustrating a drawback when an EVS is used for eye tracking of comparative example 1.
[Fig. 3]

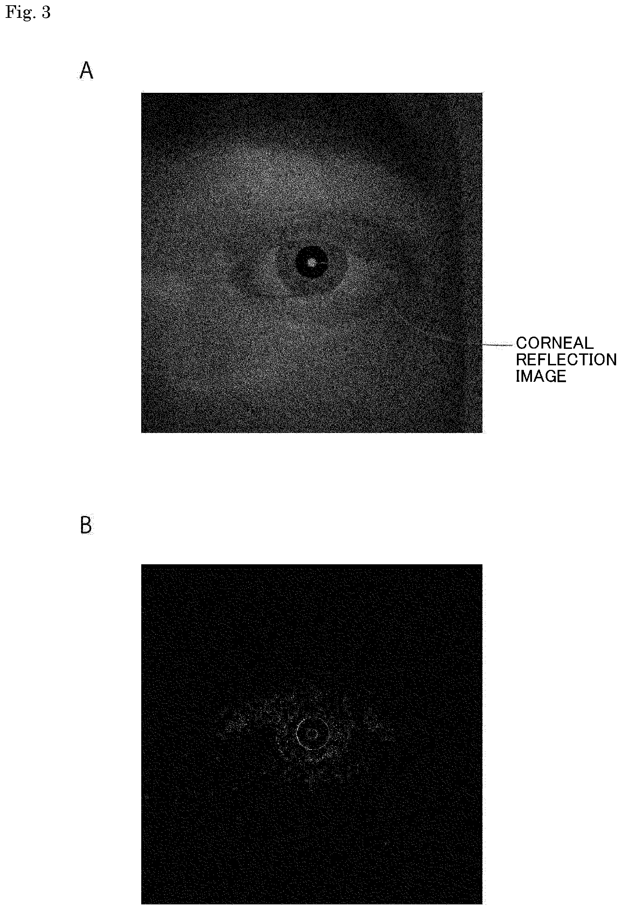

   Figs. 3A and 3B are explanatory drawings illustrating a drawback when an EVS is used for eye tracking of comparative example 2.
[Fig. 4]

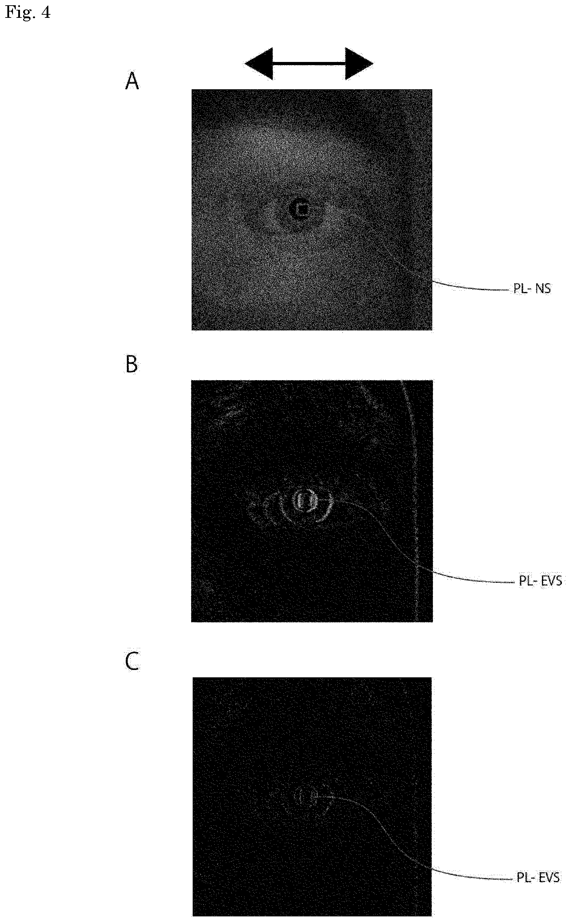

   Figs. 4A to 4C are explanatory drawings illustrating a drawback when an EVS is used for eye tracking of comparative example 3.
[Fig. 5]

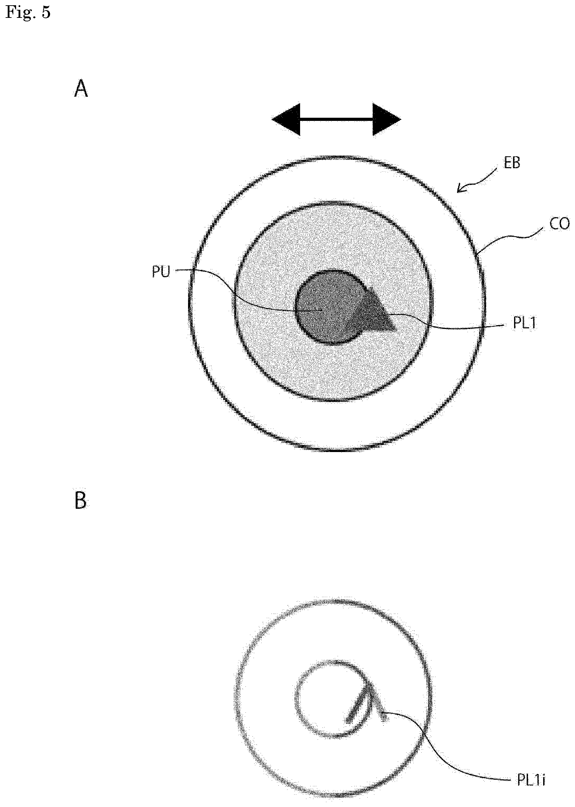

   Fig. 5A illustrates an eyeball irradiated with patterned light from a gaze detection device according to a first embodiment of the present technique. Fig. 5B illustrates an EVS image of the eyeball.
[Fig. 6]

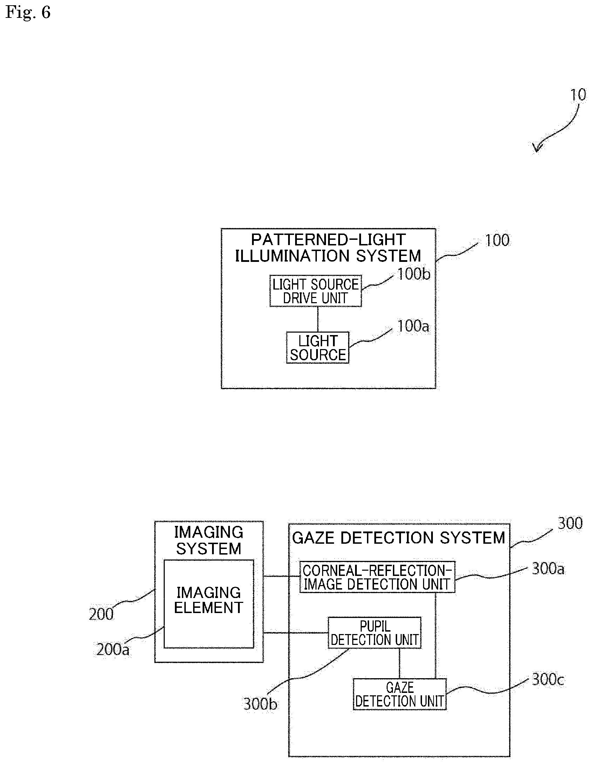

   Fig. 6 is a block diagram illustrating a function example of the gaze detection device according to the first embodiment of the present technique.
[Fig. 7]

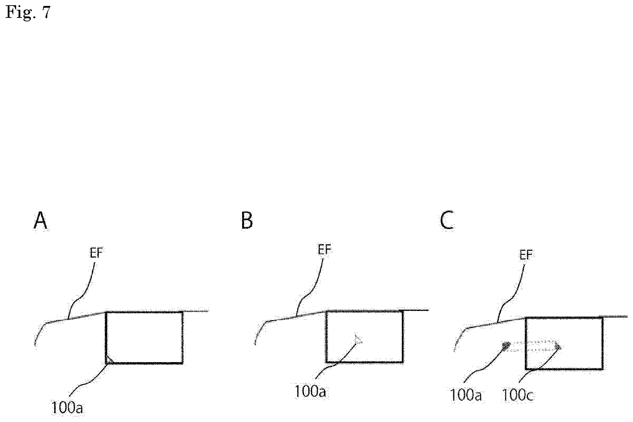

   Figs. 7A to 7C illustrate configuration examples 1 to 3 of a light source unit for a patterned-light illumination system of the gaze detection device according to the first embodiment of the present technique.
[Fig. 8]

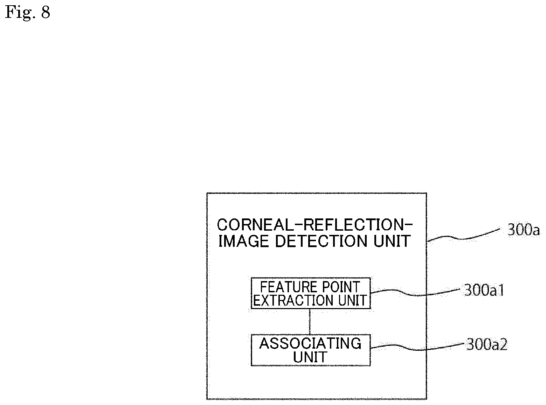

   Fig. 8 is a block diagram showing function example 1 of a corneal-reflection-image detection unit.
[Fig. 9]

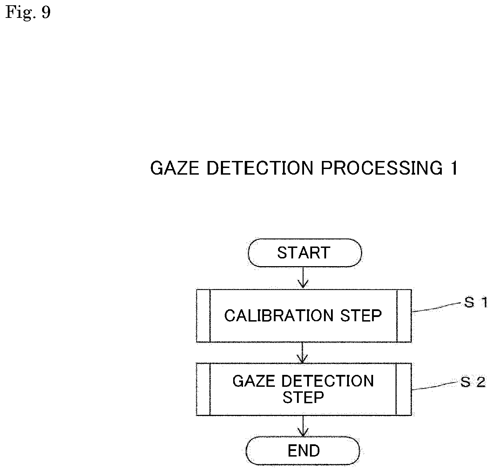

   Fig. 9 is a flowchart for describing gaze detection processing 1.
[Fig. 10]

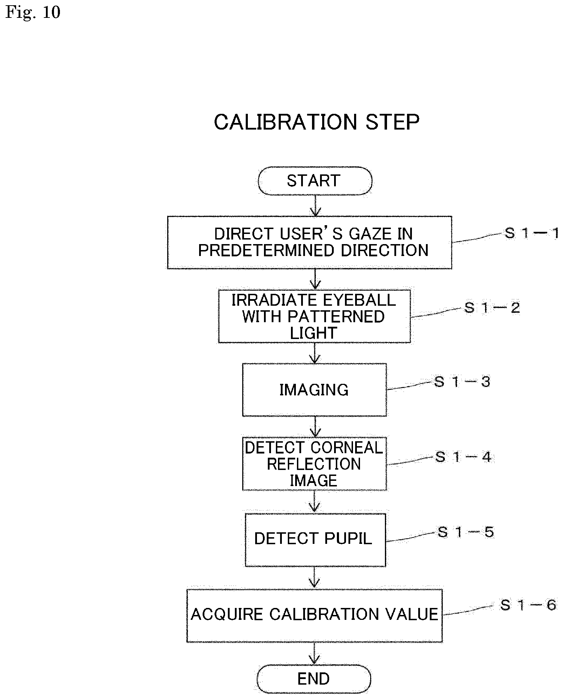

   Fig. 10 is a flowchart for describing the calibration step of Fig. 9.
[Fig. 11]

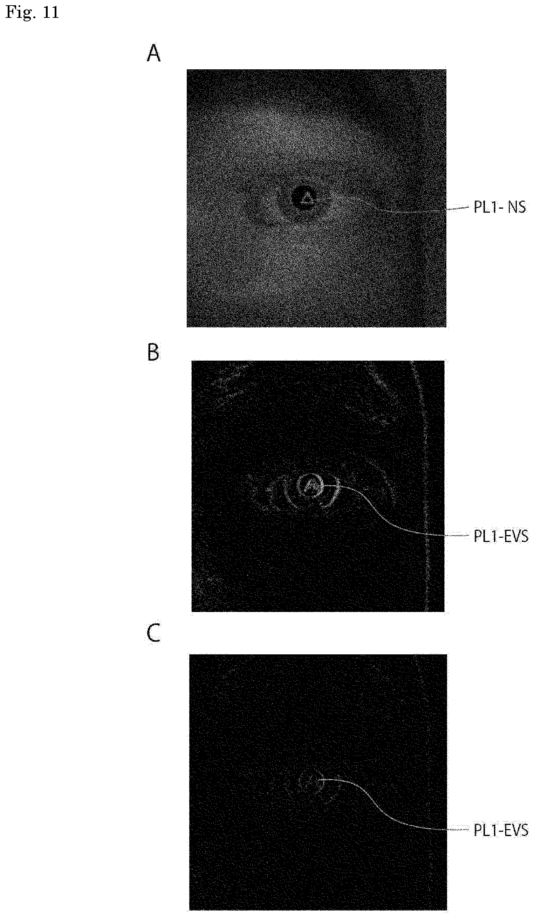

   Figs. 11A to 11C are explanatory drawings of an operation example of the gaze detection device according to the first embodiment of the present technique.
[Fig. 12]

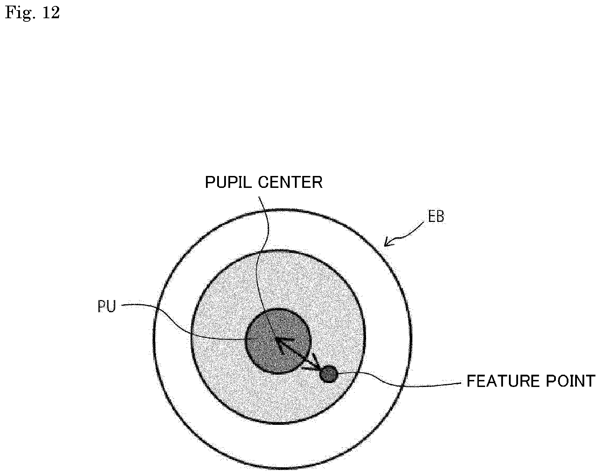

   Fig. 12 is an explanatory drawing of acquisition of a calibration value in Fig. 10.
[Fig. 13]

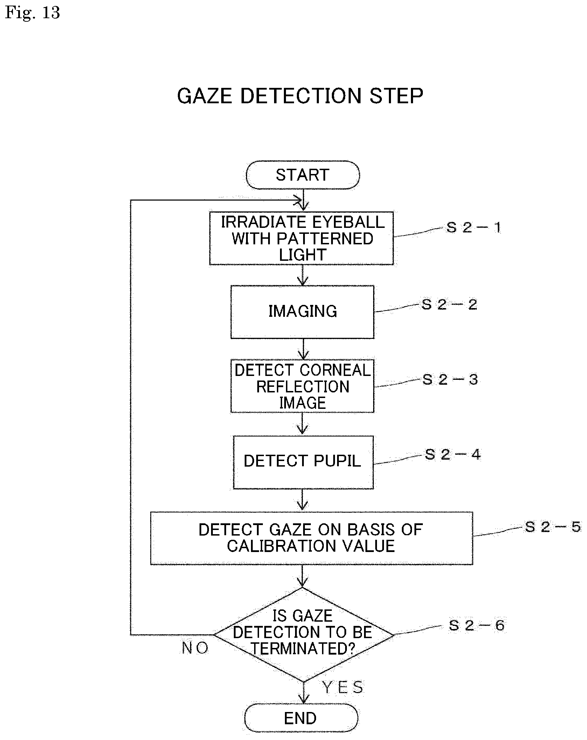

   Fig. 13 is a flowchart for describing the gaze detection step of Fig. 9.
[Fig. 14]

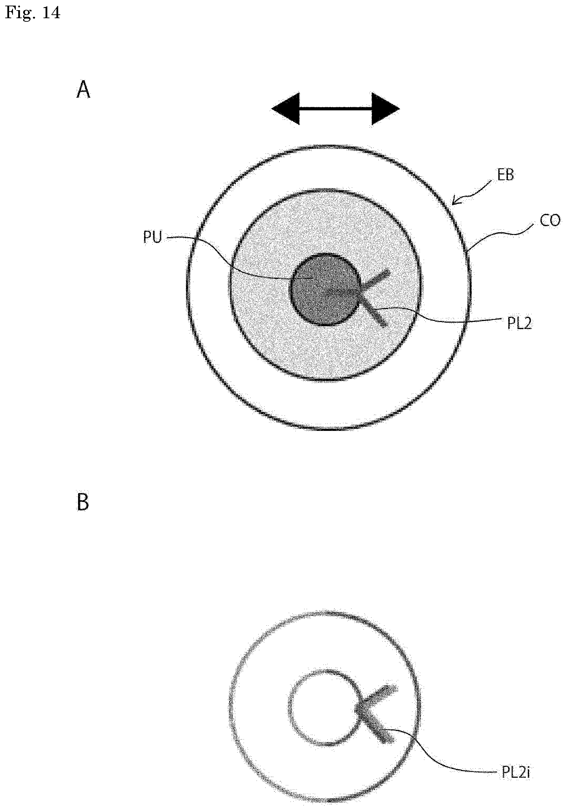

   Fig. 14A illustrates an eyeball irradiated with patterned light from a gaze detection device according to modification example 1 of the first embodiment of the present technique. Fig. 14B illustrates an EVS image of the eyeball.
[Fig. 15]

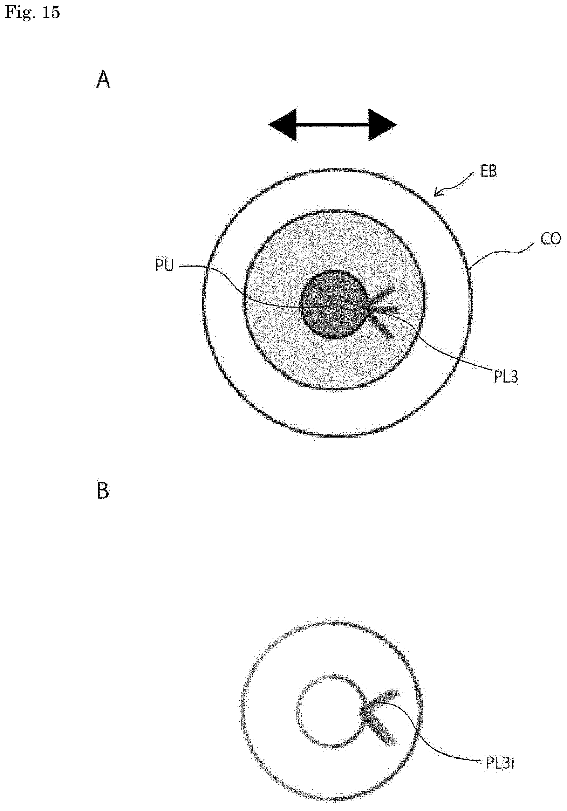

   Fig. 15A illustrates an eyeball irradiated with patterned light from a gaze detection device according to modification example 2 of the first embodiment of the present technique. Fig. 15B illustrates an EVS image of the eyeball.
[Fig. 16]

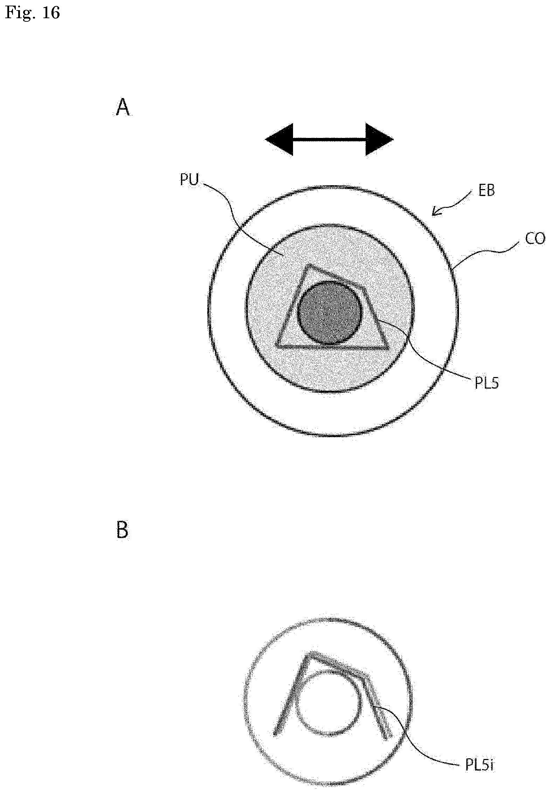

   Fig. 16A illustrates an eyeball irradiated with patterned light from a gaze detection device according to modification example 3 of the first embodiment of the present technique. Fig. 16B illustrates an EVS image of the eyeball.
[Fig. 17]

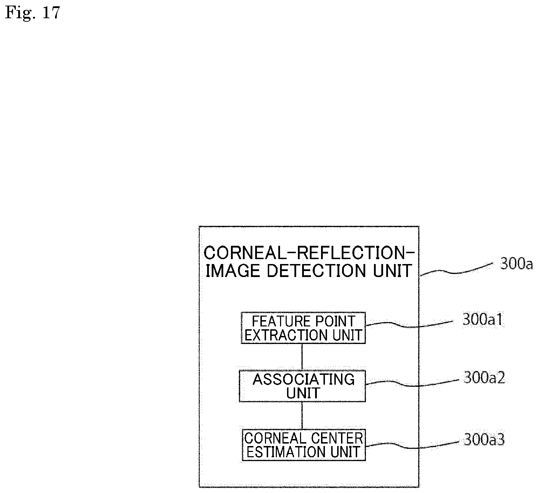

   Fig. 17 is a block diagram illustrating function example 2 of the corneal-reflection-image detection unit.
[Fig. 18]

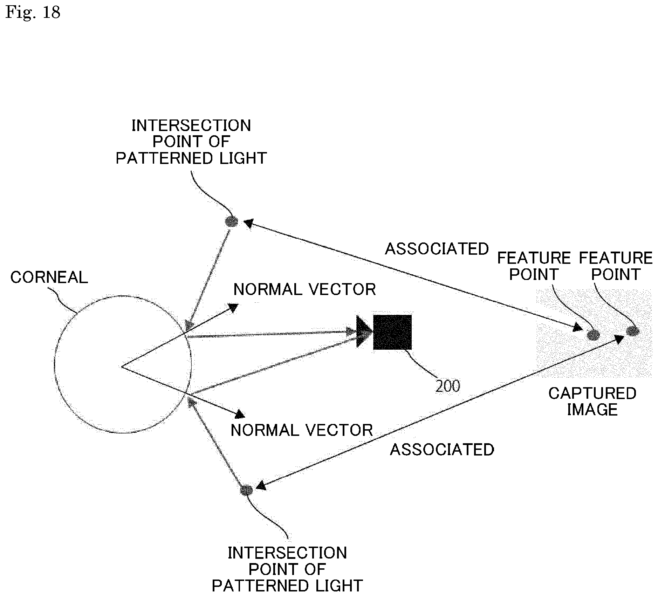

   Fig. 18 illustrates an operation example of the corneal-reflection-image detection unit in Fig. 17.
[Fig. 19]

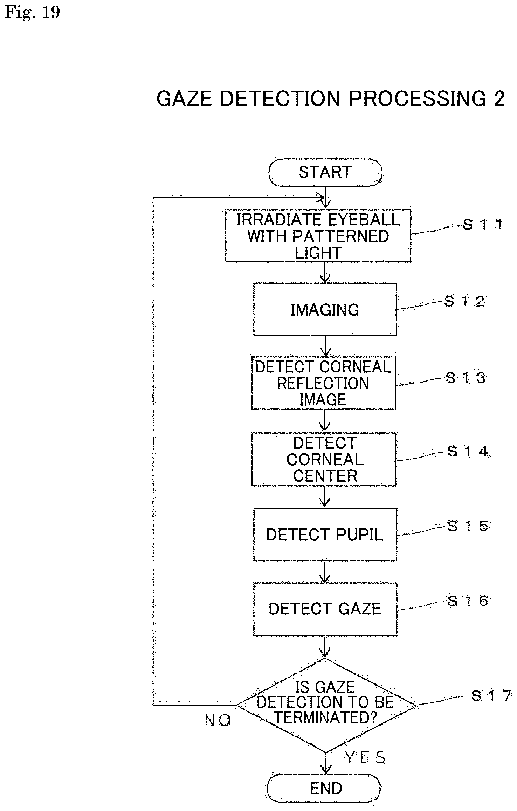

   Fig. 19 is a flowchart for describing gaze detection processing 2.
[Fig. 20]

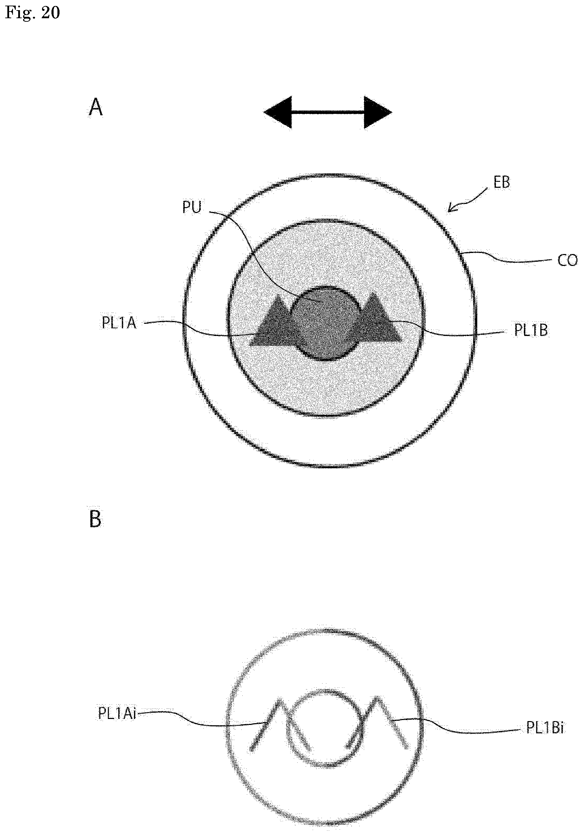

   Fig. 20A illustrates an eyeball irradiated with patterned light from a gaze detection device according to a second embodiment of the present technique. Fig. 20B illustrates an EVS image of the eyeball.
[Fig. 21]

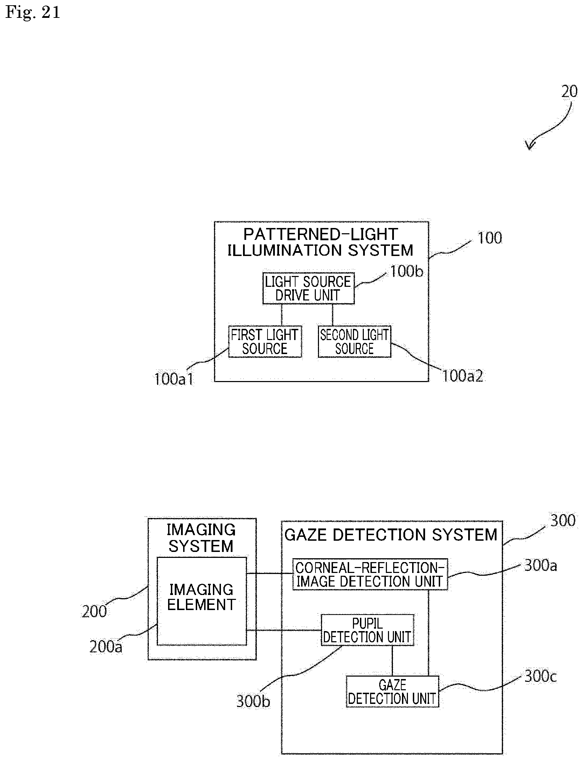

   Fig. 21 is a block diagram illustrating a function example of the gaze detection device according to the second embodiment of the present technique.
[Fig. 22]

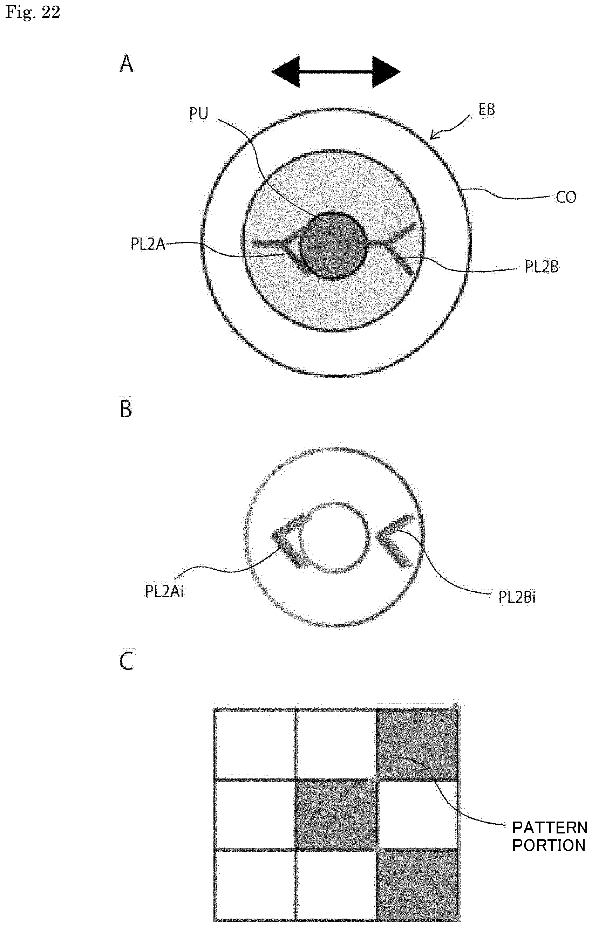

   Fig. 22A illustrates an eyeball irradiated with patterned light from a gaze detection device according to modification example 1 of the second embodiment of the present technique. Fig. 22B illustrates an EVS image of the eyeball. Fig. 22C is an explanatory drawing of a method for detecting an intersection point from the captured image.
[Fig. 23]

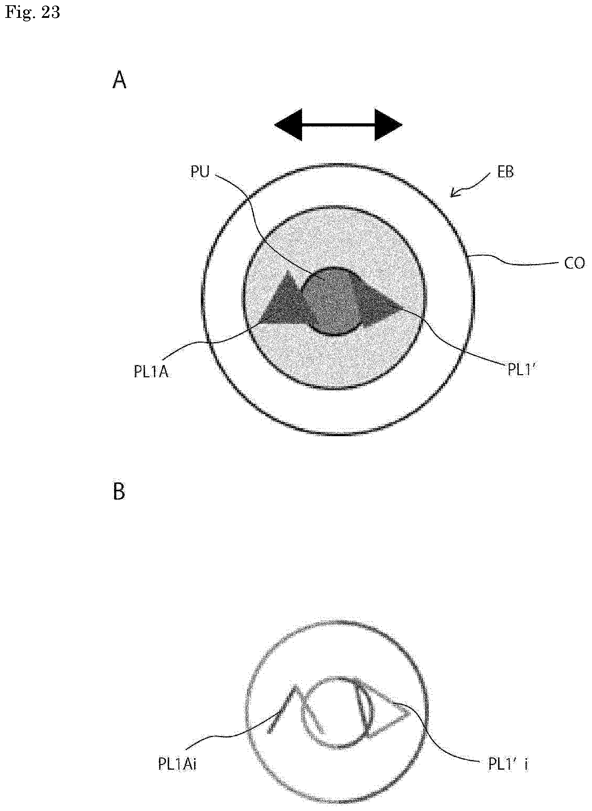

   Fig. 23A illustrates an eyeball irradiated with patterned light from a gaze detection device according to modification example 2 of the second embodiment of the present technique. Fig. 23B illustrates an EVS image of the eyeball.
[Fig. 24]

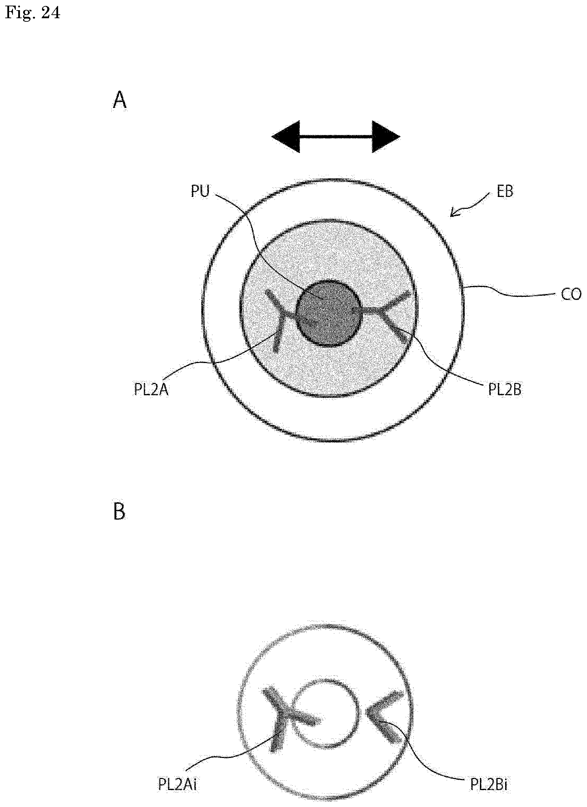

   Fig. 24A illustrates an eyeball irradiated with patterned light from a gaze detection device according to modification example 3 of the second embodiment of the present technique. Fig. 24B illustrates an EVS image of the eyeball.
[Fig. 25]

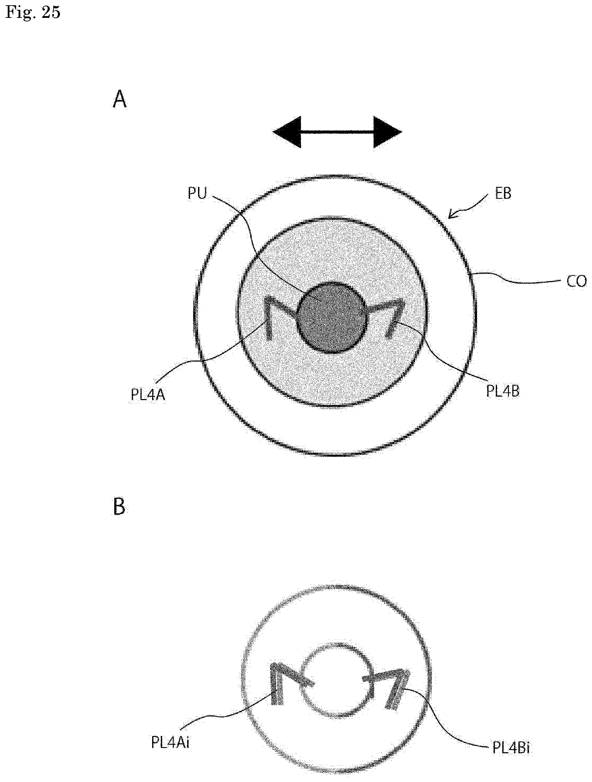

   Fig. 25A illustrates an eyeball irradiated with patterned light from a gaze detection device according to modification example 4 of the second embodiment of the present technique. Fig. 25B illustrates an EVS image of the eyeball.
[Fig. 26]

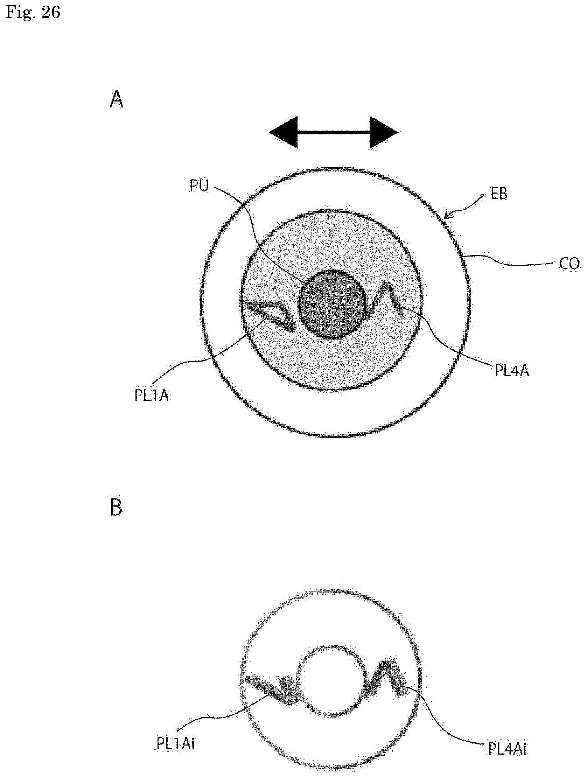

   Fig. 26A illustrates an eyeball irradiated with patterned light from a gaze detection device according to modification example 5 of the second embodiment of the present technique. Fig. 26B illustrates an EVS image of the eyeball.
[Fig. 27]

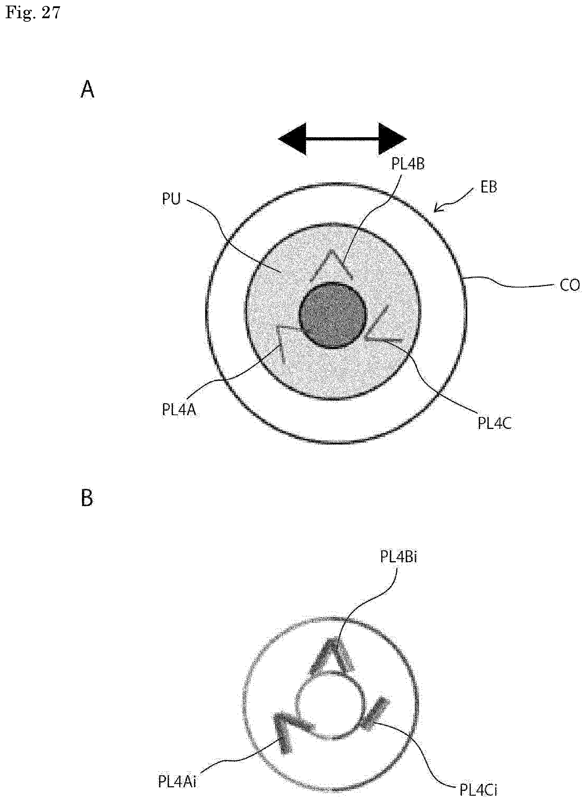

   Fig. 27A illustrates an eyeball irradiated with patterned light from a gaze detection device according to a third embodiment of the present technique. Fig. 27B illustrates an EVS image of the eyeball.
[Fig. 28]

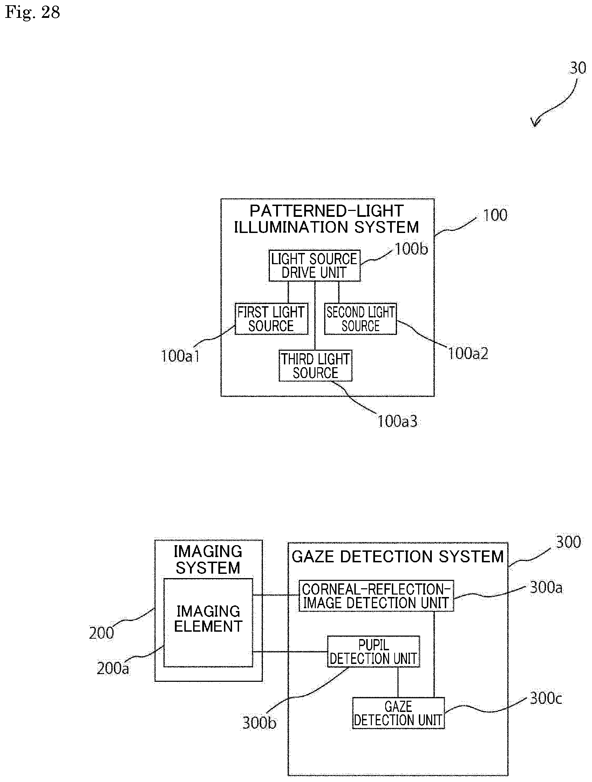

   Fig. 28 is a block diagram illustrating a function example of the gaze detection device according to the third embodiment of the present technique.
[Fig. 29]

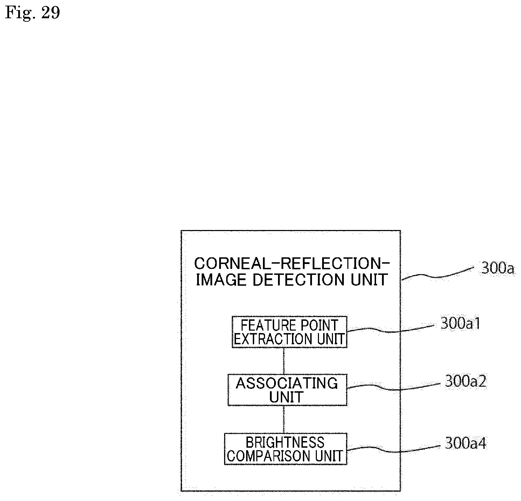

   Fig. 29 is a block diagram illustrating function example 3 of the corneal-reflection-image detection unit.
[Fig. 30]

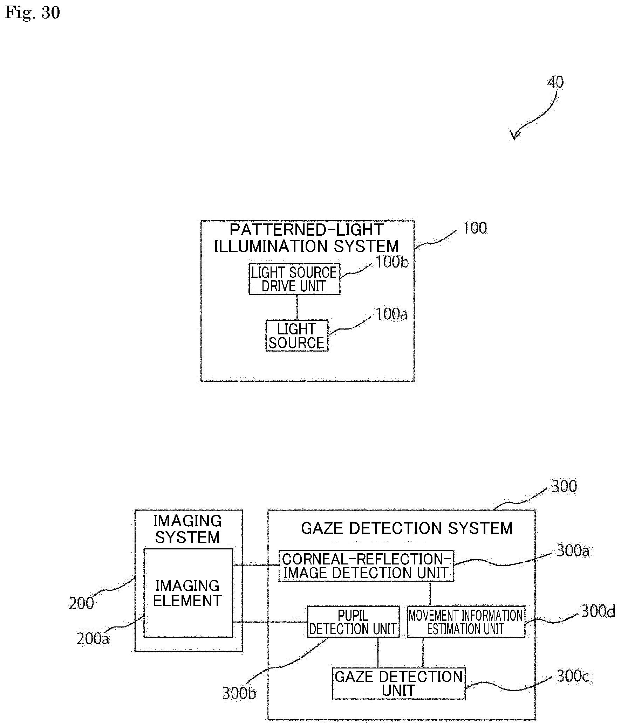

   Fig. 30 is a block diagram illustrating a function example of a gaze detection device according to a fourth embodiment of the present technique.
[Fig. 31]

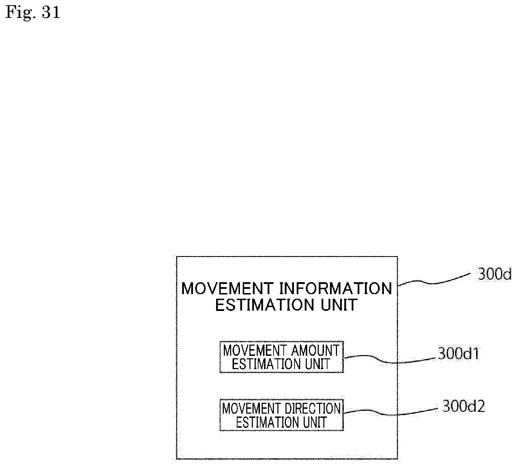

   Fig. 31 is a block diagram illustrating function example 1 of a movement information estimation unit.
[Fig. 32]

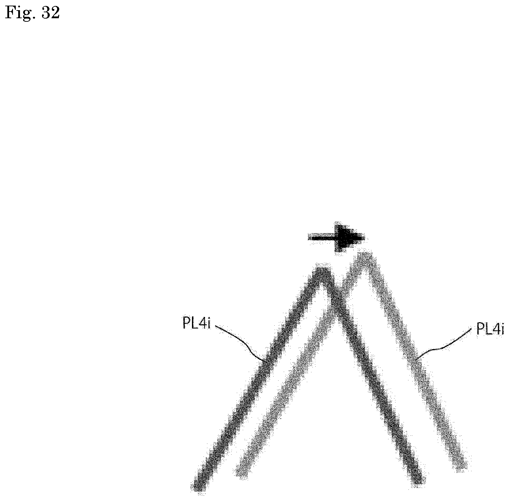

   Fig. 32 is an explanatory drawing of the principle of function example 1 of the movement information estimation unit.
[Fig. 33]

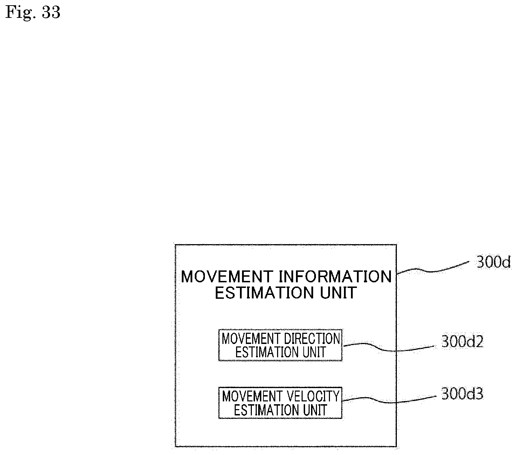

   Fig. 33 is a block diagram illustrating function example 2 of the movement information estimation unit.
[Fig. 34]

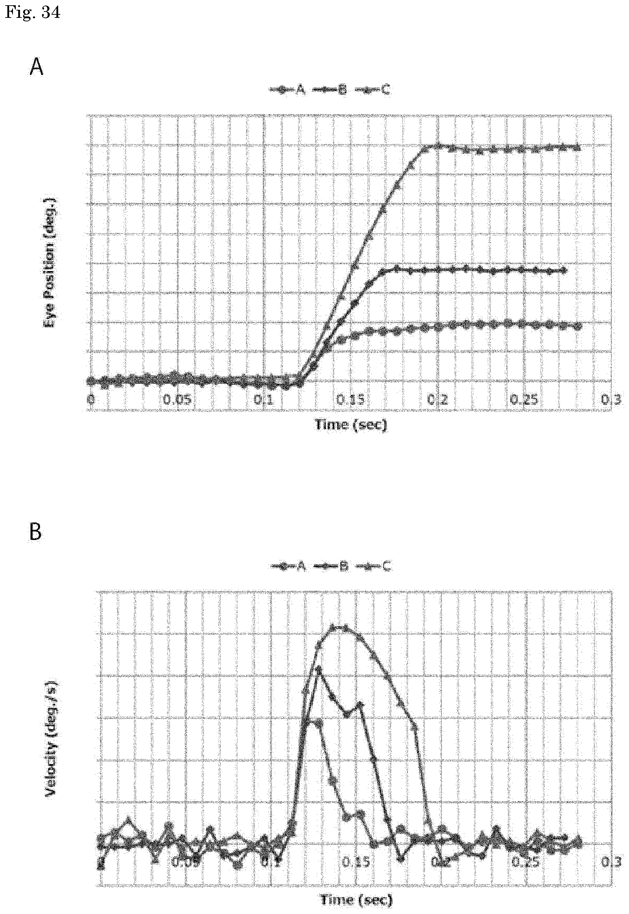

   Figs. 34A and 34B are explanatory drawings showing the principle of function example 2 of the movement information estimation unit.
[Fig. 35]

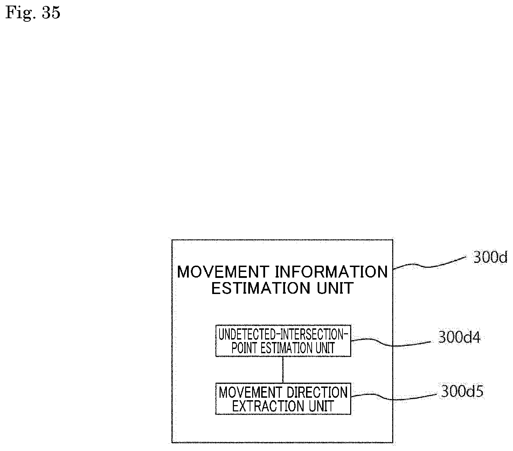

   Fig. 35 is a block diagram illustrating function example 3 of the movement information estimation unit.
[Fig. 36]

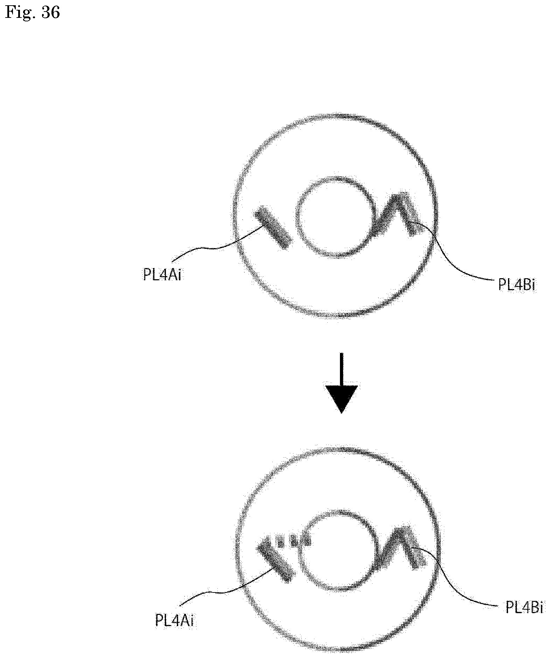

   Fig. 36 is an explanatory drawing of the principle of function example 3 of the movement information estimation unit.
[Fig. 37]

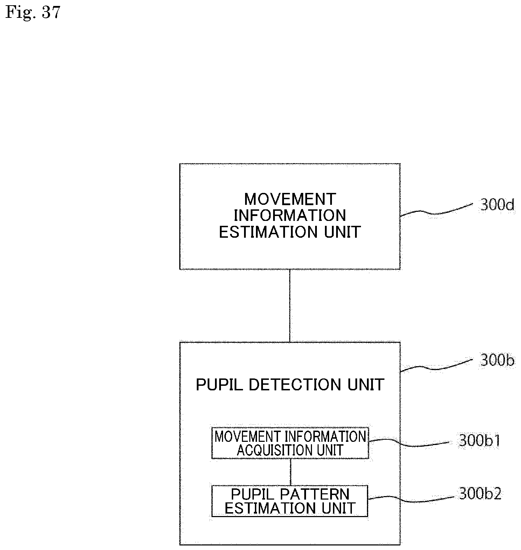

   Fig. 37 is a block diagram illustrating a function example of a pupil detection unit.
[Fig. 38]

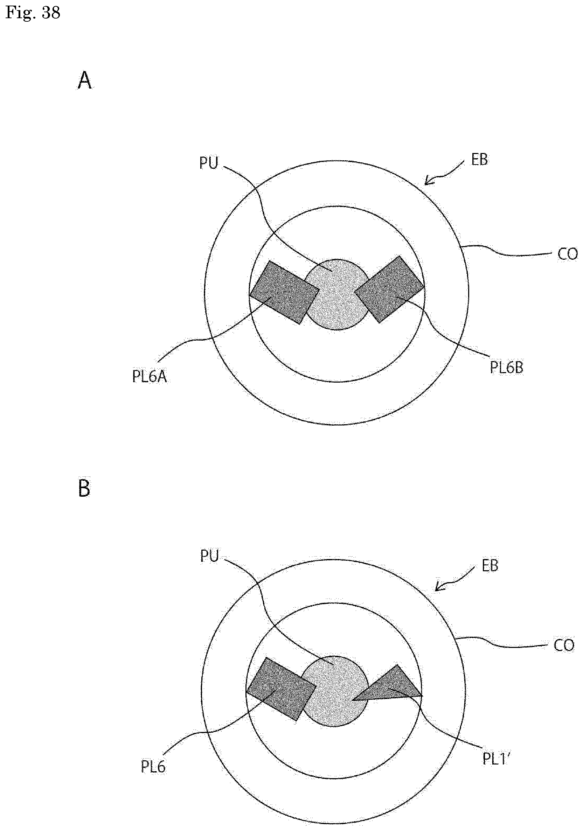

   Figs. 38A and 38B illustrate eyeballs irradiated with patterned light from gaze detection devices according to modification examples 6 and 7 of the second embodiment of the present technique.
[Fig. 39]

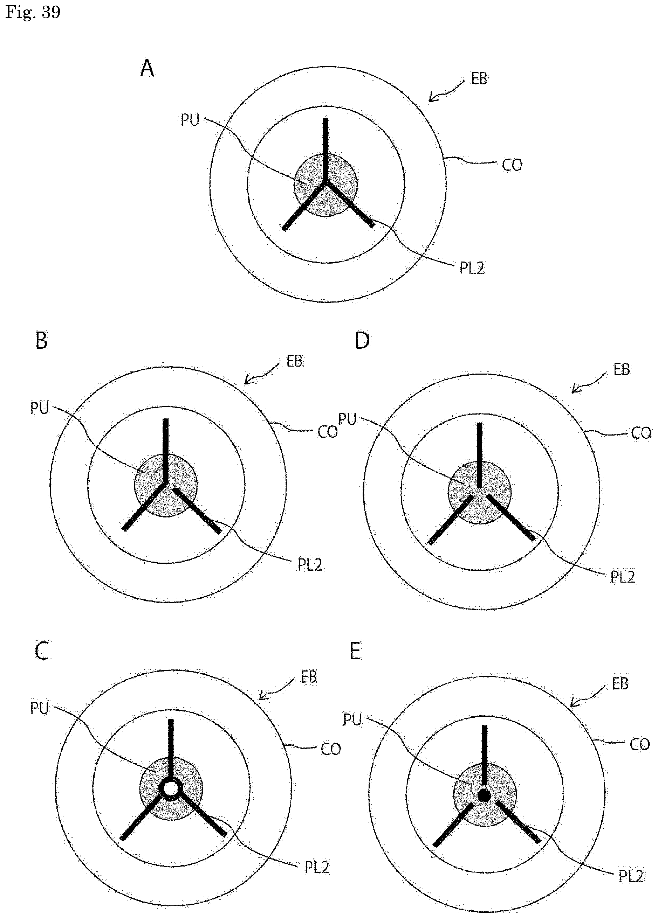

   Figs. 39A to 39E illustrate eyeballs irradiated with patterned light from gaze detection devices according to modification examples 4 to 8 of the first embodiment of the present technique.
[Fig. 40]

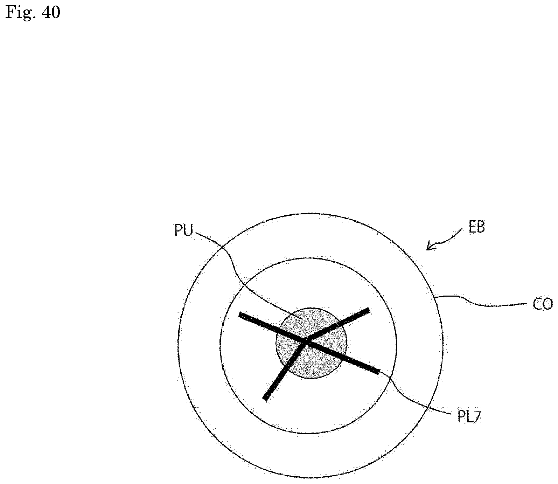

   Fig. 40 illustrates an eyeball irradiated with patterned light from a gaze detection device according to modification example 9 of the first embodiment of the present technique.
[Fig. 41]

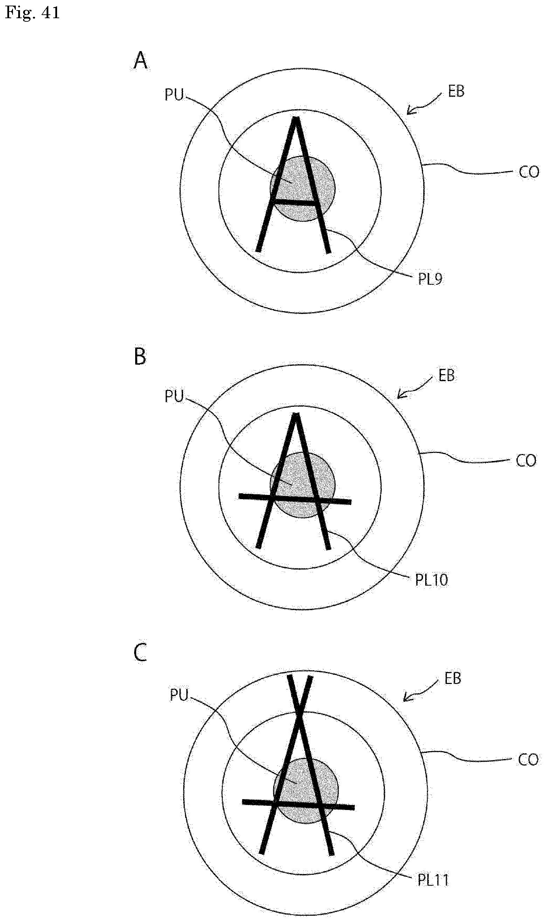

   Figs. 41A to 41C illustrate eyeballs irradiated with patterned light from gaze detection devices according to modification examples 10 to 12 of the first embodiment of the present technique.
[Fig. 42]

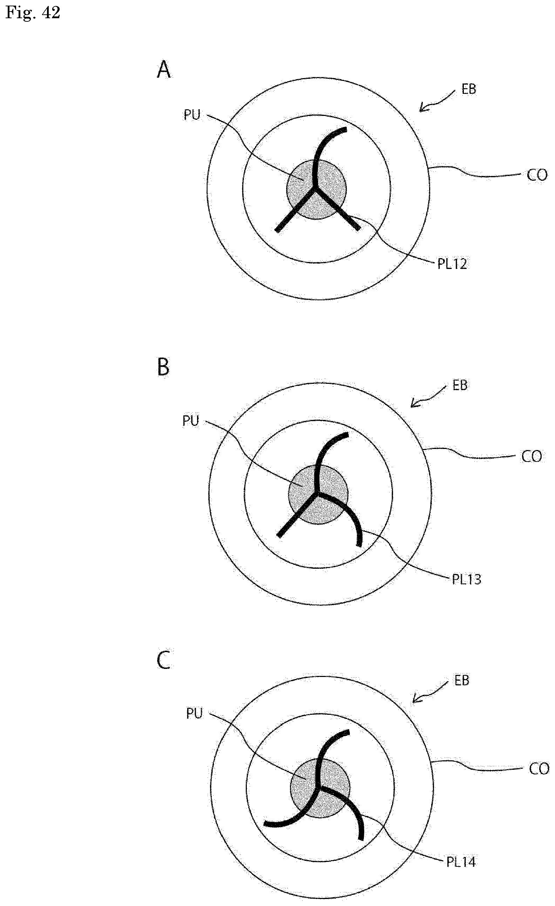

   Figs. 42A to 42C illustrate eyeballs irradiated with patterned light from gaze detection devices according to modification examples 13 to 15 of the first embodiment of the present technique.
[Fig. 43]

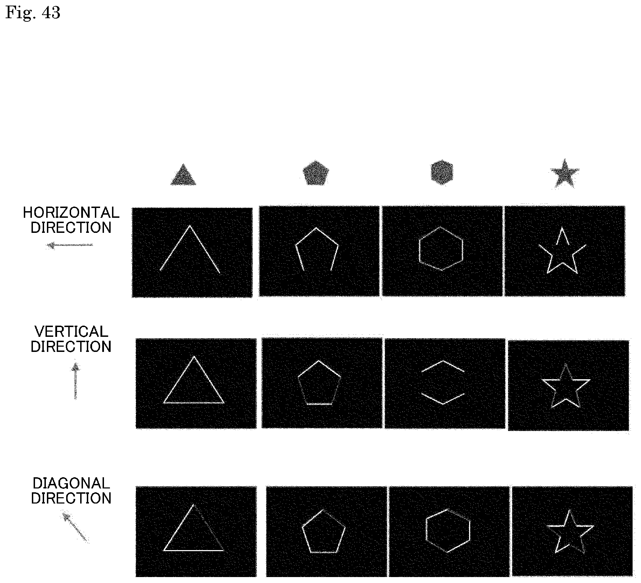

   Fig. 43 shows variation 1 of patterned light suitable for the gaze detection device according to the present technique and corneal reflection images of the patterned light.
[Fig. 44]

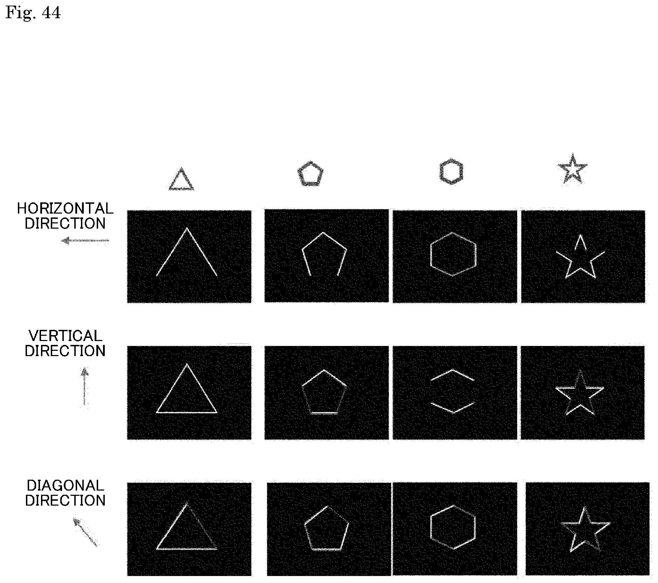

   Fig. 44 shows variation 2 of patterned light suitable for the gaze detection device according to the present technique and corneal reflection images of the patterned light.

### [Description of Embodiments]

Preferred embodiments of the present technique will be described in detail with reference to the accompanying drawings. In the present specification and the drawings, components having substantially the same functional configuration will be denoted by the same reference signs, and thus repeated descriptions thereof will be omitted. The following embodiments describe representative embodiments of the present technique, and the scope of the present technique should not be narrowly interpreted on the basis of the embodiments. Even when the present specification describes that the gaze detection device according to the present technique exhibits a plurality of advantageous effects, the gaze detection device according to the present technique only needs to exhibit at least one of the advantageous effects. The advantageous effects described in the present specification are merely exemplary and are not limited, and other advantageous effects may be obtained.

The description will be made in the following order:
0. Introduction
1. Gaze Detection Device according to First Embodiment of Present Technique
2. Gaze Detection Device according to Modification Example 1 of First Embodiment of Present Technique
3. Gaze Detection Device according to Modification Example 2 of First Embodiment of Present Technique
4. Gaze Detection Device according to Modification Example 3 of First Embodiment of Present Technique
5. Gaze Detection Device according to Second Embodiment of Present Technique
6. Gaze Detection Device according to Modification Example 1 of Second Embodiment of Present Technique
7. Gaze Detection Device according to Modification Example 2 of Second Embodiment of Present Technique
8. Gaze Detection Device according to Modification Example 3 of Second Embodiment of Present Technique
9. Gaze Detection Device according to Modification Example 4 of Second Embodiment of Present Technique
10. Gaze Detection Device according to Modification Example 5 of Second Embodiment of Present Technique
11. Gaze Detection Device according to Third Embodiment of Present Technique
12. Gaze Detection Device according to Fourth Embodiment of Present Technique
13. Gaze Detection Devices according to Modification Examples 6 and 7 of Second Embodiment of Present Technique
14. Gaze Detection Devices according to Modification Examples 4 to 8 of First Embodiment of Present Technique
15. Gaze Detection Device according to Modification Example 9 of First Embodiment of Present Technique
16. Gaze Detection Devices according to Modification Examples 10 to 12 of First Embodiment of Present Technique
17. Gaze Detection Devices according to Modification Examples 13 to 15 of First Embodiment of Present Technique
18. Variation 1 of Patterned Light
19. Variation 2 of Patterned Light
20. Other Modification Examples of present technique

### <0. Introduction>

### (Conventional eye tracking method)

A conventionally known gaze detection device (eye tracking device) detects gaze, the direction of an eyeball, by irradiating the eyeball with light (e.g., invisible light) and imaging the eyeball with an imaging system. As a first example of a conventional eye tracking method, a polynomial fitting method for a pupil and a corneal reflection image is known as illustrated in Fig. 1A. In this method, the relationship between the corneal reflection image of a light source and a pupil position is associated with a gaze by calibration.

As a second example of a conventional eye tracking method, a model estimation method for a pupil and a corneal reflection image is known as illustrated in Fig. 1B. In this method, a gaze is detected by determining a cornea center from a plurality of corneal reflection images and determining a pupil center from a pupil image.

Although conventional eye tracking in the first and second examples can obtain high accuracy, a trade-off is made between power consumption and a frame rate. To address this problem, for example, EVS (event-based vision sensor) may be used. EVSs have recently received attention as high-speed imaging element with low power consumption.

Unfortunately, if an EVS is used for eye tracking, robustness may decrease. This is because a corneal reflection image (Purkinje image) is difficult to detect. Fig. 2A shows an image of an eyeball irradiated with light from a small point source, the image being captured by an ordinary imaging element. Fig. 2B shows an EVS image of an eye ball irradiated with light from a small point source. When a small point source is used as shown in Figs. 2A and 2B, it is difficult to extract a corneal reflection image with the small point source according to a computer vision algorithm without gradation. Furthermore, a large quantity of external light such as sunlight enters a pupil as a point source, causing difficulty in identifying an eyeball.

Fig. 3A shows an image of an eyeball irradiated with light from a large point source, the image being captured by an ordinary imaging element. Fig. 3B shows an EVS image of an eye ball irradiated with light from a large point source. As shown in Figs. 3A and 3B, the light source may be upsized (for example, a circular light source having a large diameter may be used). In this case, the center of the circle may be a feature point of the circle but is not preferable because the radius of the circle is affected by ambiguity such as an eye movement or a corneal curvature.

A literature (Event-Based Kilohertz Eye Tracking using Coded Differential Lighting) proposes an approach to improvement of the detection accuracy of a corneal reflection image when an EVS is used for eye tracking. This literature describes that the detection accuracy of Purkinje is improved by blinking two adjacent LEDs, but unfortunately control is necessary for preventing the blinking LEDs from causing an overall change in brightness, and a blinking frequency depends upon the bandwidth of the EVS. An overall change in brightness may cause simultaneous firing of events, resulting in latency.

Thus, an eyeball may be irradiated with patterned light to increase feature points, which may cause the following problems:
· An eyeball movement changes viewing of a pattern.
· An eyeball movement causes at least a part of a pattern to disappear.

These considerably reduce the robustness of detection.

A simulation example of eye tracking performed by irradiating an eyeball with patterned light PL like a rectangular frame will be described below. Fig. 4A shows an image of an eyeball irradiated with the patterned light PL like a rectangular frame, the image being captured by an ordinary imaging element NS. Fig. 4B shows an image of an eyeball irradiated with the patterned light PL like a rectangular frame when moving horizontally with a relatively high velocity, the image being captured by EVS. Fig. 4C shows an image of an eyeball irradiated with the patterned light PL like a rectangular frame when moving horizontally with a relatively low velocity, the image being captured by EVS. In Fig. 4A, an image PL-NS of reflected light of the patterned light PL like a rectangular frame can be clearly confirmed. In Fig. 4B, the rectangular frame appears with double lines in an image PL-EVS of reflected light of the patterned light PL. The lines are thick and thus cause ambiguity in fitting between a light source and the reflected light. Also in Fig. 4C, the rectangular frame appears with double lines in an image PL-EVS of reflected light of the patterned light PL, and the brightness hardly changes horizontally. Thus, the shape of the patterned light PL is considerably deformed.

Hence, through earnest examination, the inventors have developed a gaze detection device according to the present technique as a gaze detection device (eye tracking device) that can suppress reduction in detection accuracy regardless of the imaging method of an imaging system by designing the shape of patterned light. The gaze detection device according to the present technique is quite effective particularly when an EVS is used for an imaging system.

### <1. Gaze Detection Device according to First Embodiment of Present Technique>

### <<Configuration of Gaze Detection Device>>

Fig. 5A illustrates an eyeball EB irradiated with patterned light from a gaze detection device 10 according to a first embodiment of the present technique. Fig. 5B illustrates an EVS image of the eyeball EB. Fig. 6 is a block diagram illustrating a function example of a gaze detection device 10 according to the first embodiment of the present technique.

The gaze detection device 10 is mounted on, for example, an HMD (Head Mounted Display) and is used for eye tracking (gaze detection). As illustrated in Fig. 6, the gaze detection device 10 includes a patterned-light illumination system 100, an imaging system 200, and a gaze detection system 300. For example, the gaze detection device 10 is supported by an HMD support structure (e.g., an eye glass frame) mounted on a user's head. The HMD displays an image superimposed on a user's field of view. The HMD controls the display of an image on the basis of, for example, the output (user's line of sight) of the gaze detection device 10. The support structure of the HMD may include a voice output device that provides voice information to a user.

### (Patterned-Light Illumination System)

The patterned-light illumination system 100 irradiates the eyeball EB of a user with patterned light PL1 (see Fig. 5A). For example, the patterned light PL1 is triangular in shape. The patterned-light illumination system 100 includes, for example, a light source 100a and a light source drive unit 100b (light source driver) (see Fig. 6). For example, the light source 100a emits invisible light (e.g., infrared light). The light source 100a is, for example, a point source, a line source, or a surface source. As the light source 100a, for example, an LED (Light Emitting Diode), an OLED (Organic Light Emitting Diode), or an LD (Laser Diode) can be used. In Fig. 5A and other drawings, reference character CO denotes a cornea, and reference character PU denotes a pupil.

The patterned light PL1 has a shape along a reference pattern BP (e.g., a triangle) having at least three lines not parallel to one another, the reference pattern BP having an intersection point of at least two of the at least three lines. The at least three lines that constitute the reference pattern BP and are not parallel to one another include, for example, a line constituting a part of a figure, a line constituting a part of the outline of a figure, and a line constituting a part of a character. The at least three lines may include a straight line and a curve or at least a partially broken line as well as a solid line. The patterned light PL1 may have a pattern completely identical to the reference pattern BP or may have a pattern nearly identical to the reference pattern BP. In this case, "a pattern nearly identical to the reference pattern BP" includes, for example, a pattern obtained by removing at least one intersection point of the reference pattern BP and a pattern obtained by replacing at least one intersection point of the reference pattern BP with a figure. In this pattern, the patterned light PL1 completely agrees with the reference pattern BP. The shape of the reference pattern may be the shape of the light source or the shape of an optical element that shapes light from the light source.

Each of the at least three (e.g., three) lines of the patterned light PL1 intersects at least one (e.g., two) of the other lines. To be specific, each of the at least three lines of the patterned light PL1 intersects at least two (e.g., two) of the other lines. For example, the reference pattern BP has at least three intersection points (e.g., three intersection points) not located on the same straight line. More specifically, the reference pattern BP and the patterned light PL1 are each shaped like a triangle (e.g., an isosceles triangle, specifically a regular triangle) with one side extending nearly horizontally and the height direction nearly along the vertical direction.

Figs. 7A to 7C illustrate configuration examples 1 to 3 of a light source unit for the patterned-light illumination system 100 of the gaze detection device 10 according to the first embodiment of the present technique. In configuration example 1 of Fig. 7A, the light source 100a having the same shape (e.g., a triangle) as the patterned light PL1 is provided on an eyeglass frame EF (for example, the rim of the eyeglass frame EF). In configuration example 2 of Fig. 7B, the translucent light source 100a (e.g., uLED) having the same shape (e.g., a triangle) as the patterned light PL1 is provided in transparent glass or resin (for example, transparent glass or resin fit into the rim of the eyeglass frame EF) attached into the eyeglass frame EF. In configuration example 3 of Fig. 7C, an optical element (e.g., a diffraction element) having the same shape as the patterned light PL1 is provided in transparent glass or resin (for example, transparent glass or resin fit into the rim of the eyeglass frame EF) attached into the eyeglass frame EF, and the optical element is irradiated with light from the light source 100a. In other words, the optical element shapes light from the light source 100a into the shape of the patterned light PL1. In the configuration examples of Figs. 7A to 7C, the eyeglass frame may be a rimless frame.

The light source drive unit 100b includes, for example, circuit elements such as a transistor, a capacitor, and a resistor. The light source drive unit 100b generates a driving signal (e.g., a pulse signal) and applies the signal to the light source 100a.

### (Imaging System)

The imaging system 200 captures an image of the eyeball EB irradiated with the patterned light PL1 and outputs the captured image to the gaze detection system 300. As illustrated in Fig. 6, the imaging system 200 is, for example, a camera including an imaging element 200a. The imaging system 200 may include, in addition to the imaging element 200a, a condensing element (e.g., a condenser lens or a condenser mirror) that condenses the patterned light PL1 onto the imaging element 200a after the patterned light PL1 is emitted to the eyeball EB from the patterned-light illumination system 100 and is reflected by the eyeball EB, and a signal processing unit that processes the output signal of the imaging element 200a. Furthermore, the imaging system 200 may include an imaging element (e.g., an ordinary image sensor) for detecting a pupil.

The imaging element 200a is, for example, an EVS (event-based vision sensor). The EVS has a plurality of pixels in a two-dimensional array. The EVS is a vision sensor that detects a change in the brightness of each pixel and outputs the brightness value (pixel value) of only the pixel having changed in brightness in combination with time information and a vision sensor that achieves a high-speed operation and low power consumption. The EVS includes, for example, a light receiving circuit having a photoelectric conversion element (e.g., a photodiode having sensitivity in an infrared region) for each pixel, an amplifier, and a comparator. In the EVS, for example, incident light is converted into an electric signal by the light receiving circuit, is amplified by the amplifier, and is divided to be output as a positive change signal (positive event) and a negative change signal (negative event) depending upon the threshold value of brightness. The output signal of the EVS is output as a captured image through the signal processing unit. The EVS is also referred to as "event sensor" or "event vision sensor."

### (Gaze Detection System)

The gaze detection system 300 includes, for example, a corneal-reflection-image detection unit 300a, a pupil detection unit 300b, and a gaze detection unit 300c. The gaze detection system 300 may further include a storage unit (e.g., memory). The gaze detection system 300 is implemented by, for example, hardware such as a CPU and a chip set.

The corneal-reflection-image detection unit 300a detects a feature point of a corneal reflection image of the patterned light PL1 from a captured image of the imaging system 200. Specifically, the corneal-reflection-image detection unit 300a detects a corneal reflection image of the patterned light PL1 from a captured image of the imaging system 200 by image processing (e.g., edge detection, outline extraction, and shade detection) and extracts a feature point of the corneal reflection image. Furthermore, the corneal-reflection-image detection unit 300a associates an intersection point of the reference pattern BP (an intersection point of the patterned light PL1) with the feature point of the corneal reflection image of the patterned light PL1.

Fig. 8 is a block diagram showing function example 1 of the corneal-reflection-image detection unit. As shown in Fig. 8, the corneal-reflection-image detection unit 300a includes a feature point extraction unit 300a1 and an associating unit 300a2. The feature point extraction unit 300a1 extracts a feature point of the corneal reflection image of the patterned light PL1 by, for example, corner detection or a DNN (Deep Neural Network). The associating unit 300a2 associates a feature point of the corneal reflection image of the patterned light PL1 and an intersection point of the reference pattern BP (an intersection point of the patterned light PL1) on the basis of information about an angle and/or a direction near the feature point in the captured image of the imaging system 200, stores the associating result in the storage unit (e.g., memory), and/or outputs the associating result to the gaze detection unit 300c.

Specifically, in Fig. 5A, only the pixel value of a pixel having changed in brightness is output in the patterned light PL1 from the EVS when the eyeball EB moves, for example, in the horizontal direction, so that the EVS captures an image as illustrated in Fig. 5B. As a supplementary explanation, a corneal reflection image PL1i (an output signal of the EVS) composed of two oblique side portions in a reversed V shape appears in the image captured by the EVS in response to a change in brightness in the patterned light PL1 when the eyeball EB moves in the horizontal direction. In this case, the feature point extraction unit 300a1 extracts the intersection point of the two oblique side portions of the corneal reflection image PL1i as a feature point of the corneal reflection image of the patterned light PL1. Moreover, the associating unit 300a2 associates the feature point and the intersection point of the patterned light PL1, from information about an angle and/or a direction (e.g., an acute angle or an upward direction) near the feature point (e.g., the intersection point of the two oblique side portions).

In the example of Fig. 5A, the eyeball EB moves in a direction (e.g., the horizontal direction) parallel to a line of the reference pattern BP. When the eyeball EB moves in a direction not parallel to any one of the three non-parallel lines of the reference pattern BP, the three non-parallel lines of the reference pattern BP (patterned light PL1) appear in the captured image of the EVS. In this case, three feature points (intersection points) may be detected in the corneal reflection image of the reference pattern BP.

For example, by using image processing (e.g., edge detection, outline extraction, and shade detection), the pupil detection unit 300b extracts a pupil from the captured image of the imaging element for detecting a pupil, calculates the center position of the pupil in the coordinate system of the captured image, and stores the calculation result in the storage unit (e.g., memory), and/or outputs the calculation result to the gaze detection unit 300c.

For example, the gaze detection unit 300c detects gaze, the direction of the eyeball EB, on the basis of the detection result obtained by the pupil detection unit 300b and the detection result obtained by the corneal-reflection-image detection unit 300a on the basis of the captured image of the imaging system 200.

### <<Gaze Detection Processing 1>>

Referring to Figs. 9 to 13, gaze detection processing 1 performed by, for example, the gaze detection device 10 will be described below. Fig. 9 is a flowchart for describing the gaze detection processing 1.

In step S1, a calibration step is performed. The calibration step will be specifically described later.

In step S2, a gaze detection step is performed. The gaze detection step will be specifically described later. After step S2 is performed, the flow is terminated.

### (Step S1: calibration step)

Referring to the flowchart of Fig. 10, the calibration step of Fig. 9 will be described below.

In first step S1-1, the user's gaze is directed in a predetermined direction. Specifically, a user is notified (for example, through display by an HMD or voice by the voice output device) to look at a predetermined gaze point (e.g., the front), so that the user's gaze is directed in the predetermined direction (e.g., to the front).

In next step S1-2, the eyeball EB is irradiated with the patterned light PL1. Specifically, the light source 100a is driven by the light source drive unit 100b.

In next step S1-3, imaging is performed. Specifically, an image of the eyeball EB irradiated with the patterned light PL1 is captured by the imaging element (e.g., an ordinary image sensor) for detecting a pupil.

In next steps S1-4, a corneal reflection image is detected. Specifically, the corneal-reflection-image detection unit 300a extracts the feature point of the corneal reflection image of the patterned light PL1 from the image captured in step S1-3, and associates the feature point and the intersection point of the patterned light PL1.

In next step S1-5, a pupil is detected. Specifically, the pupil detection unit 300b extracts a pupil PU from the captured image of the imaging element for detecting a pupil and detects the center position of the pupil.

In next step S1-6, a calibration value is acquired. Specifically, the positional relationship (see Fig. 12) between the center of the pupil detected in step S1-5 and the corneal reflection image of the intersection point of the patterned light PL1 is associated with the gaze direction (e.g., to the front) and is acquired as a calibration value, the corneal reflection image being detected in step S1-4, and then the calibration value is stored in the storage unit (e.g., memory).

The accuracy of the calibration value can be increased by performing the series of steps S1-1 to S1-6 several times to obtain multiple calibration values and using the mean value or the median value of the calibration values. In this case, the predetermined direction in step S1-1 in each time may be the same direction or different directions.

### (Step S2: Gaze Detection Step)

Referring to the flowchart of Fig. 13, the gaze detection step of Fig. 9 will be described below.

In first step S2-1, the eyeball EB is irradiated with the patterned light PL1. Specifically, the light source 100a is driven by the light source drive unit 100b.

In next step S2-2, imaging is performed. Specifically, the imaging element 200a (EVS) of the imaging system 200 captures an image of the eyeball EB irradiated with the patterned light PL1 and outputs the captured image (see Figs. 11B and 11C). Fig. 11A shows an image of the eyeball EB irradiated with the patterned light PL1, the image being captured by an ordinary imaging element NS. Fig. 11B shows an EVS image of the eyeball irradiated with the patterned light PL1 when the eyeball moves horizontally with a relatively high velocity. Fig. 11C shows an EVS image of the eyeball irradiated with the patterned light PL1 when the eyeball moves horizontally with a relatively low velocity. In Fig. 11A, an image PL1-NS of reflected light of the patterned light PL1 can be clearly confirmed. In Fig. 11B, although two oblique sides appear thickly as double lines in an image PL1-EVS of reflected light of the patterned light PL1, a corner composed of the two oblique sides and the intersection point thereof can be detected all the time, enabling robust detection of the eyeball EB moving at high velocity. Also in Fig. 11C, although two oblique sides appear thickly as double lines in an image PL1-EVS of reflected light of the patterned light PL1 and brightness hardly changes in the horizontal direction, a corner composed of the two oblique sides and the intersection point thereof can be detected all the time, enabling robust detection of the eyeball EB moving at low velocity.

In next steps S2-3, a corneal reflection image is detected. Specifically, the corneal-reflection-image detection unit 300a extracts the feature point of the corneal reflection image of the patterned light PL1 and associates the feature point and the intersection point of the patterned light PL1.

In next step S2-4, a pupil is detected. Specifically, the pupil detection unit 300b extracts the pupil PU from the captured image of the imaging element for detecting a pupil and detects the center position of the pupil.

In next step S2-5, a gaze is detected on the basis of a calibration value. Specifically, the gaze direction is determined on the basis of the positional relationship between the center of the pupil detected in step S2-4 and the corneal reflection image of each intersection point of the patterned light PL1, the corneal reflection image being detected in step S2-3, and the calibration value that is acquired in the calibration step (step S1) and is stored in the storage unit.

In final step S2-6, whether to terminate the gaze detection is determined. When the gaze detection device 10 is turned off, the termination is acknowledged. When the gaze detection device 10 is turned on, the termination is rejected to perform the gaze detection again (steps S2-1 to S2-5).

### <<Effect of Gaze Detection Device>>

Effects of the gaze detection device 10 will be described below.

The gaze detection device 10 according to the first embodiment of the present technique includes the patterned-light illumination system 100 that irradiates the eyeball EB with the patterned light PL1, the imaging system 200 that captures an image of the eyeball EB irradiated with the patterned light PL1, and the gaze detection system 300 that detects a gaze as the direction of the eyeball EB on the basis of the captured image of the imaging system 200. The patterned light PL1 has a shape along the reference pattern BP having at least three lines not parallel to one another, the reference pattern BP having an intersection point of at least two of the at least three lines.

In this case, for example, if the imaging method of the imaging system 200 is the event-based vision method, at least two non-parallel lines of the patterned light PL1 can be detected in the captured image regardless of the direction of movement of the eyeball EB irradiated with the patterned light PL1, so that a feature point (e.g., an intersection point) formed by the at least two lines can be extracted.

Consequently, according to the gaze detection device 10, the gaze detection device capable of suppressing reduction in detection accuracy can be provided regardless of the imaging method of the imaging system 200. If the imaging method of the imaging system 200 is not the event-based vision method (for example, in the case of an ordinary image sensor), the gaze detection device 10 can detect at least three non-parallel lines of the patterned light PL1 in the captured image, thereby extracting a feature point (e.g., an intersection point) formed by the at least three lines. Also in this case, the gaze detection device capable of suppressing reduction in detection accuracy can be provided.

Each of the at least three (e.g., three) lines of the reference pattern BP crosses at least one (e.g., two) of the other lines. Thus, the patterned light PL1 can have quite a simple shape that allows detection of a feature point regardless of the imaging method. Hence, the light source and the optical element that are used for generating the patterned light PL1 can have quite simple shapes.

Each of the at least three (e.g., three) lines of the reference pattern BP crosses at least two of the other lines. Thus, the patterned light PL1 can have a simple shape that allows detection of a feature point regardless of the imaging method. Hence, the light source and the optical element that are used for generating the patterned light PL1 can have simple shapes.

The reference pattern BP has at least three intersection points on the same straight line. Thus, at least one feature point (e.g., one intersection point) of the patterned light PL1 can be detected in the captured image regardless of the direction of movement of the eyeball EB irradiated with the patterned light PL1, thereby improving the detection accuracy.

The gaze detection system 300 includes the corneal-reflection-image detection unit 300a that detects a feature point (e.g., an intersection point) of the corneal reflection image of the patterned light PL1 from the captured image of the imaging system 200. This enables robust detection of a movement of the eyeball EB.

The corneal-reflection-image detection unit 300a associates the intersection point of the reference pattern BP and the feature point on the basis of information about an angle and/or a direction near the feature point (e.g., the intersection point of the reference pattern BP) of the corneal reflection image of the reference pattern BP in the captured image of the imaging system 200. This can associate the intersection point and the feature point with high accuracy.

The gaze detection system 300 detects a gaze on the basis of the captured image of the imaging system 200 and the detection result of the corneal-reflection-image detection unit 300a. This can detect a gaze with high accuracy.

The patterned-light illumination system 100 may include the light source 100a that emits patterned light. Thus, the light source 100a can have a simple shape and thus is easy to manufacture.

The patterned-light illumination system 100 may include the light source 100a and the optical element that shapes light from the light source 100a to generate the patterned light PL1. Thus, the optical element can have a simple shape and thus is easy to manufacture.

The imaging system 200 includes the EVS. This enables high-speed imaging with low power consumption.

### <2. Gaze Detection Device according to Modification Example 1 of First Embodiment of Present Technique>

Fig. 14A illustrates an eyeball irradiated with patterned light from a gaze detection device according to modification example 1 of the first embodiment of the present technique. Fig. 14B illustrates an EVS image of the eyeball.

As illustrated in Figs. 14A and 14B, the gaze detection device according to modification example 1 of the first embodiment has the same configuration as the gaze detection device 10 according to the first embodiment except that patterned light PL2 emitted from the patterned-light illumination system 100 to the eyeball EB has a different shape from the patterned light PL1.

In modification example 1, the reference pattern BP has at least three non-parallel lines (e.g., three lines) that cross at the same intersection point and extend radially from the intersection point. Specifically, in modification example 1, the reference pattern BP has a horizontally oriented Y shape (a Y shape rotated 90°). In this pattern, the patterned light PL2 completely agrees with the reference pattern BP.

Also in modification example 1, for example, when the eyeball EB moves in the horizontal direction (lateral direction), a corneal reflection image PL2i of the patterned light PL2 does not have any horizontal lines with constant brightness in the EVS image but has two oblique lines with a change in brightness. The intersection point of the two oblique lines is extracted as a feature point, enabling gaze detection with high accuracy.

According to the gaze detection device of modification example 1, the same effect as the gaze detection device 10 according to the first embodiment can be obtained, and the reference pattern BP has the single intersection point (the intersection point of the patterned light PL2). Thus, the corneal reflection image and the intersection point can be easily associated with each other, enabling gaze detection at higher speed.

### <3. Gaze Detection Device according to Modification Example 2 of First Embodiment of Present Technique>

Fig. 15A illustrates an eyeball irradiated with patterned light from a gaze detection device according to modification example 2 of the first embodiment of the present technique. Fig. 15B illustrates an EVS image of the eyeball.

As illustrated in Figs. 15A and 15B, the gaze detection device according to modification example 2 of the first embodiment has the same configuration as the gaze detection device 10 according to the first embodiment except that patterned light PL3 emitted from the patterned-light illumination system 100 to the eyeball EB has a different shape from the patterned light PL1.

In modification example 2, the reference pattern BP has at least three non-parallel lines that cross at the same intersection point and form consecutive acute angles around the intersection point. In modification example 2, for example, the reference pattern BP is shaped like a horizontally oriented arrow. In this pattern, the patterned light PL3 completely agrees with the reference pattern BP.

Also in modification example 2, for example, when the eyeball EB moves in the horizontal direction, a corneal reflection image PL3i of the patterned light PL3 does not have any horizontal lines with constant brightness in the EVS image but has two oblique lines with a change in brightness. The intersection point of the two oblique lines is extracted as a feature point, enabling gaze detection with high accuracy.

According to the gaze detection device of modification example 2, the same effect as the gaze detection device 10 according to the first embodiment can be obtained, and the reference pattern BP has the single intersection point (the intersection point of the patterned light PL3). Thus, the corneal reflection image and the intersection point can be easily associated with each other, enabling gaze detection at higher speed.

### <4. Gaze Detection Device according to Modification Example 3 of First Embodiment of Present Technique>

### <<Configuration of Gaze Detection Device>>

Fig. 16A illustrates an eyeball irradiated with patterned light PL5 from a gaze detection device according to modification example 3 of the first embodiment of the present technique. Fig. 16B illustrates an EVS image of the eyeball.

As illustrated in Figs. 16A and 16B, the gaze detection device according to modification example 3 of the first embodiment has the same configuration as the gaze detection device 10 according to the first embodiment except that patterned light PL5 emitted from the patterned-light illumination system 100 to the eyeball EB has a different shape from the patterned light PL1.

In modification example 3, the reference pattern BP has at least three (e.g., four) non-parallel lines, each crossing at least two (e.g., two) of the other lines. In modification example 3, for example, the reference pattern BP is shaped like a quadrilateral having four non-parallel sides, one of which is nearly parallel to the horizontal direction. For example, it is assumed that the size of the reference pattern BP is greater than or equal to the size of the pupil PU.

In this case, the patterned light PL5 completely agrees with the reference pattern BP.

In modification example 3, for example, when the eyeball EB moves in the horizontal direction, a corneal reflection image PL5i of the patterned light PL5 does not have any horizontal lines with constant brightness in the EVS image but has three oblique lines with a change in brightness (see Fig. 16B). Two intersection points formed by the three oblique lines are extracted as feature points, enabling gaze detection with high accuracy.

Fig. 17 is a block diagram showing function example 2 of the corneal-reflection-image detection unit. In the gaze detection device according to modification example 3, as shown in Fig. 17, the corneal-reflection-image detection unit 300a includes the feature point extraction unit 300a1, the associating unit 300a2, and a corneal center estimation unit 300a3.

Fig. 18 illustrates an operation example of the corneal-reflection-image detection unit of Fig. 17. The feature point extraction unit 300a1 extracts a plurality of feature points of the corneal reflection image PL5i of the patterned light PL5 from the EVS image. The associating unit 300a2 associates the feature points extracted by the feature point extraction unit 300a1 and the intersection points of the patterned light PL5. From the plurality of feature points that are extracted by the feature point extraction unit 300a1 and are associated with the plurality of intersection points by the associating unit 300a2, the corneal center estimation unit 300a3 determines the reflecting points of the plurality of corresponding intersection points of the patterned light PL5 on the corneal, determines normal vectors passing through the respective reflecting points, estimates the intersection point of the normal vectors as a corneal center (the center of a corneal CO) in the coordinate system of the captured image, and outputs the estimation result to the gaze detection unit 300c.

For example, by using image processing (e.g., edge detection, outline extraction, and shade detection), the pupil detection unit 300b extracts a pupil from the captured image of the imaging element for detecting a pupil, calculates the center position of the pupil in the coordinate system of the captured image, and outputs the calculation result to the gaze detection unit 300c.

The gaze detection unit 300c determines a gaze direction on the basis of the corneal center as the estimation result of the corneal center estimation unit 300a3 and the pupil center as the detection result of the pupil detection unit 300b.

### <<Gaze Detection Processing 2>>

Referring to Fig. 19, gaze detection processing 2 performed by, for example, the gaze detection device according to modification example 3 will be described below. Fig. 19 is a flowchart for describing the gaze detection processing 2.

In first step S11, the eyeball EB is irradiated with patterned light. Specifically, the light source 100a is driven by the light source drive unit 100b.

In next step S12, imaging is performed. Specifically, the imaging element 200a (EVS) of the imaging system 200 captures an image of the eyeball EB irradiated with the patterned light and outputs the captured image.

In next steps S13, a corneal reflection image is detected. Specifically, the corneal-reflection-image detection unit 300a extracts multiple feature points of the corneal reflection image of the patterned light from the captured image, and associates corneal reflection images and the intersection points of the patterned light.

In next steps S14, the corneal center is estimated. Specifically, the corneal-reflection-image detection unit 300a calculates the corneal center of the corneal CO on the basis of the feature points of the corneal reflection image of the patterned light.

In next step S15, a pupil is detected. Specifically, the pupil detection unit 300b extracts the pupil PU from the captured image of the imaging element for detecting a pupil and detects the center position of the pupil.

In next step S16, a gaze is detected. Specifically, a gaze direction is determined on the basis of the corneal center estimated in step S14 and the pupil center detected in step S15.

In final step S17, whether to terminate the gaze detection is determined. When the gaze detection device according to modification example 3 is turned off, the termination is acknowledged. When the gaze detection device is turned on, the termination is rejected to perform the gaze detection again (steps S11 to S16).

### <<Effect of Gaze Detection Device>>

According to the gaze detection device of modification example 3, at least two feature points of the corneal reflection image of the patterned light PL5 can be detected all the time, so that a gaze can be detected by the gaze detection processing 2 that does not require calibration. Furthermore, the patterned light PL5 is relatively large, so that a feature point of the corneal reflection image is easily detected.

### <5. Gaze Detection Device according to Second Embodiment of Present Technique>

Fig. 20A illustrates an eyeball irradiated with patterned light PL1 from a gaze detection device according to a second embodiment of the present technique. Fig. 20B illustrates an EVS image of the eyeball. Fig. 21 is a block diagram illustrating a function example of a gaze detection device 20 according to the second embodiment of the present technique.

The gaze detection device 20 according to the second embodiment has the same configuration as the gaze detection device 10 according to the first embodiment except that a reference pattern BP has multiple (e.g., two) patterns (e.g., triangular patterns), each including at least two (e.g., three) lines crossing each other.

In the gaze detection device 20, the reference pattern BP includes at least two (e.g., two) patterns that are identical in shape (including a similar figure, same as below) and are oriented in the same direction. In this case, patterned light corresponding to the reference pattern BP completely agrees with the reference pattern BP. Specifically, the patterned light corresponding to the reference pattern BP includes, for example, multiple (e.g., two) first and second pattern portions PL1A and PL1B that are identical in shape and are oriented in the same direction as illustrated in Fig. 20A. In this case, the patterns of the reference pattern BP and the pattern portions of the patterned light are triangular in shape with one side extending nearly horizontally.

As illustrated in Fig. 20B, for example, a corneal reflection image PL1Ai of the first pattern portion PL1A and a corneal reflection image PL1Bi of the second pattern portion PL1B appear in an EVS image when an eyeball EB moves horizontally. The corneal reflection images each have a reversed V shape with one intersection point.

In the gaze detection device 20, a patterned-light illumination system 100 includes first and second light sources 100a1 and 100a2 as illustrated in Fig. 21. The first light source 100a1 is used for generating the first pattern portion PL1A. The second light source 100a2 is used for generating the second pattern portion PL1B. The first and second light sources 100a1 and 100a2 are driven by a light source drive unit 100b.

Also in the gaze detection device 20, gaze detection processing 2 is performed. At this point, a feature point (an intersection point of the first pattern portion PL1A) of the corneal reflection image PL1Ai and a feature point (an intersection point of the second pattern portion PL1B) of the corneal reflection image PL1Bi are extracted, and then the corneal center is calculated using the feature points.

According to the gaze detection device 20, at least two feature points of the corneal reflection image of the patterned light can be detected all the time, so that a gaze can be detected by the gaze detection processing 2 that does not require calibration.

In the gaze detection device 20, the patterned-light illumination system 100 may irradiate the eyeball EB only with portion of patterned light (a pattern portion where a feature point is detectable), the portion corresponding to the pattern where an intersection point is detectable among the multiple patterns of the reference pattern BP. As a supplementary explanation, when the total amount of light emission from the light source unit is kept constant, irradiation of patterned light divided into multiple pattern portions may result in lower brightness in each of the pattern portions, causing lower contrast and difficulty in obtaining signals. Thus, the contrast can be increased and signals are easily obtained by reducing the number of divisions of patterned light (when patterned light is not divided, the number of divisions is 1). Whether an intersection point is detectable in the multiple patterns of the reference pattern BP can be determined by the estimation result (e.g., the direction of movement of the eyeball EB) of a movement information estimation unit 300d, which will be described later. The above-mentioned selective irradiation method of patterned light is also applicable to gaze detection devices according to other embodiments and modification examples.

### <6. Gaze Detection Device according to Modification Example 1 of Second Embodiment of Present Technique>

Fig. 22A illustrates an eyeball irradiated with patterned light from a gaze detection device according to modification example 1 of the second embodiment of the present technique. Fig. 22B illustrates an EVS image of the eyeball. Fig. 22C is an explanatory drawing of a method for detecting an intersection point from the captured image.

The gaze detection device according to modification example 1 of the second embodiment has the same configuration as the gaze detection device 20 according to the second embodiment except that patterned light emitted to the eyeball EB has first and second pattern portions PL2A and PL2B, each having a horizontally oriented Y shape (a Y shape rotated 90°).

As illustrated in Fig. 22B, for example, a corneal reflection image PL2Ai of the first pattern portion PL2A and a corneal reflection image PL2Bi of the second pattern portion PL2B appear in an EVS image when the eyeball EB moves horizontally. The corneal reflection images each have a horizontally oriented V shape (a V shape rotated 90°) with one intersection point.

Also in the gaze detection device according to modification example 1 of the second embodiment, gaze detection processing 2 is performed. At this point, the corneal-reflection-image detection unit 300a extracts a feature point (an intersection point of the first pattern portion PL2A) of the corneal reflection image PL2Ai and a feature point (an intersection point of the second pattern portion PL2B) of the corneal reflection image PL2Bi and calculates the corneal center by using the feature points.

According to the gaze detection device of modification example 1 of the second embodiment, at least two feature points of the corneal reflection image of the patterned light can be detected all the time, so that a gaze can be detected by the gaze detection processing 2 that does not require calibration. Even when the pattern portions are relatively small, a large acute angle can be obtained, so that a feature point (e.g., an intersection point) is easily detected (see Fig. 22C).

### <7. Gaze Detection Device according to Modification Example 2 of Second Embodiment of Present Technique>

Fig. 23A illustrates an eyeball irradiated with patterned light from a gaze detection device according to modification example 2 of the second embodiment of the present technique. Fig. 23B illustrates an EVS image of the eyeball.

As illustrated in Fig. 23A, the gaze detection device according to modification example 2 of the second embodiment has the same configuration as the gaze detection device 20 according to the second embodiment except that patterned light emitted to the eyeball EB has first and second pattern portions PL1A and PL1' in different shapes. The first pattern portion PL1Ais triangular in shape with one side extending nearly horizontally. The second pattern portion PL1' is shaped like a triangle dissimilar to the first pattern portion PL1A and has all the sides not parallel to any one of the sides of the first pattern portion PL1A.

As illustrated in Fig. 23B, for example, a corneal reflection image PL1Ai of the first pattern portion PL1A and a corneal reflection image PL1'i of the second pattern portion PL1' appear in an EVS image when the eyeball EB moves horizontally. The corneal reflection image PL1Ai has a reversed V shape, and the corneal reflection image PL1'i is triangular in shape (nearly identical in shape to the second pattern portion PL1').

Also in the gaze detection device according to modification example 2 of the second embodiment, gaze detection processing 2 is performed. At this point, the corneal-reflection-image detection unit 300a extracts a feature point (an intersection point of the first pattern portion PL1A) of the corneal reflection image PL1Ai and a feature point (an intersection point of the second pattern portion PL1') of the corneal reflection image PL1' and calculates the corneal center by using the feature points.

According to the gaze detection device of modification example 2 of the second embodiment, at least two feature points of the corneal reflection image of the patterned light can be detected all the time, so that a gaze can be detected by the gaze detection processing 2 that does not require calibration. Moreover, multiple feature points of the corneal reflection image can be detected, enabling gaze detection with higher accuracy.

### <8. Gaze Detection Device according to Modification Example 3 of Second Embodiment of Present Technique>

Fig. 24A illustrates an eyeball irradiated with patterned light from a gaze detection device according to modification example 3 of the second embodiment of the present technique. Fig. 24B illustrates an EVS image of the eyeball.

As illustrated in Fig. 24A, the gaze detection device according to modification example 3 of the second embodiment has the same configuration as the gaze detection device 20 according to the second embodiment except that patterned light emitted to the eyeball EB has first and second pattern portions PL2A and PL2B that are identical in shape and are oriented in different directions. The first pattern portion PL2A has a Y shape rotated by an acute angle or an obtuse angle (e.g., an obtuse angle). The second pattern portion PL2B has a horizontally oriented Y shape (a Y shape rotated 90°). In other words, the second pattern portion PL2B has three straight lines constituting the Y shape such that the straight lines are not parallel to any one of three straight lines constituting the Y shape of the first pattern portion PL2A.

As illustrated in Fig. 24B, for example, a corneal reflection image PL2Ai of the first pattern portion PL2A and a corneal reflection image PL2Bi of the second pattern portion PL2B appear in an EVS image when the eyeball EB moves horizontally. The corneal reflection image PL1Ai has a Y shape rotated by an obtuse angle (nearly identical in shape to the first pattern portion PL2A). The corneal reflection image PL2Bi has a horizontally oriented V shape (a V shape rotated 90°).

Also in the gaze detection device according to modification example 3 of the second embodiment, gaze detection processing 2 is performed. At this point, the corneal-reflection-image detection unit 300a extracts a feature point (an intersection point of the first pattern portion PL2A) of the corneal reflection image PL2Ai and a feature point (an intersection point of the second pattern portion PL2B) of the corneal reflection image PL2Bi and calculates the corneal center by using the feature points.

According to the gaze detection device of modification example 3 of the second embodiment, at least two feature points of the corneal reflection image of the patterned light can be detected all the time, so that a gaze can be detected by the gaze detection processing 2 that does not require calibration.

### <9. Gaze Detection Device according to Modification Example 4 of Second Embodiment of Present Technique>

Fig. 25A illustrates an eyeball irradiated with patterned light from a gaze detection device according to modification example 4 of the second embodiment of the present technique. Fig. 25B illustrates an EVS image of the eyeball.

As illustrated in Fig. 25A, the gaze detection device according to modification example 4 of the second embodiment has the same configuration as the gaze detection device 20 according to the second embodiment except that patterned light emitted to the eyeball EB has first and second pattern portions PL4A and PL4B that are identical in shape and are oriented in different directions. The first pattern portion PL4A has a V shape rotated by an acute angle or an obtuse angle (e.g., an obtuse angle). The second pattern portion PL4B has a V shape rotated by an acute angle or an obtuse angle (e.g., an obtuse angle) and oriented in a different direction from the first pattern portion PL4A. In other words, the second pattern portion PL4B has two straight lines constituting the V shape such that the straight lines are not parallel to any one of two straight lines constituting the V shape of the first pattern portion PL4A.

As illustrated in Fig. 25B, for example, a corneal reflection image PL4Ai of the first pattern portion PL4A and a corneal reflection image PL4Bi of the second pattern portion PL4B appear in an EVS image when an eyeball EB moves horizontally. The corneal reflection image PL4Ai is nearly identical in shape to the first pattern portion PL4A. The corneal reflection image PL4Bi is nearly identical in shape to the second pattern portion PL4B.

In the gaze detection device according to modification example 4 of the second embodiment, gaze detection processing 1 is performed. At this point, the corneal-reflection-image detection unit 300a extracts a feature point (an intersection point of the first pattern portion PL4A) of the corneal reflection image PL4Ai and/or a feature point (an intersection point of the second pattern portion PL4B) of the corneal reflection image PL4Bi.

According to the gaze detection device of modification example 4 of the second embodiment, at least one feature point of the corneal reflection image can be extracted all the time even when patterned light has quite a simple shape, so that a gaze can be detected all the time.

### <10. Gaze Detection Device according to Modification Example 5 of Second Embodiment of Present Technique>

Fig. 26A illustrates an eyeball irradiated with patterned light from a gaze detection device according to modification example 5 of the second embodiment of the present technique. Fig. 26B illustrates an EVS image of the eyeball.

As illustrated in Fig. 26A, the gaze detection device according to modification example 5 of the second embodiment has the same configuration as the gaze detection device 20 according to the second embodiment except that patterned light emitted to the eyeball EB has first and second pattern portions PL1A and PL4A in different shapes. The first pattern portion PL1A is nearly triangular in shape with one side extending nearly horizontally. The second pattern portion PL4A has a reversed V shape. The lines of the second pattern portion PL4A are not parallel to any one of the three sides of the first pattern portion PL1A.

As illustrated in Fig. 26B, for example, a corneal reflection image PL1Ai of the first pattern portion PL1A and a corneal reflection image PL4Ai of the second pattern portion PL4A appear in an EVS image when the eyeball EB moves horizontally. The corneal reflection image PL1Ai is shaped like the first pattern portion PL1A having a missing horizontal line. The corneal reflection image PL4Ai is nearly identical in shape to the second pattern portion PL4A.

Also in the gaze detection device according to modification example 5 of the second embodiment, gaze detection processing 2 is performed. At this point, the corneal-reflection-image detection unit 300a extracts a feature point (an intersection point of the first pattern portion PL1A) of the corneal reflection image PL1Ai and a feature point (an intersection point of the second pattern portion PL4A) of the corneal reflection image PL4Ai and calculates the corneal center by using the feature points.

According to the gaze detection device of modification example 5 of the second embodiment, at least two feature points of the corneal reflection image of the patterned light can be detected all the time, so that a gaze can be detected by the gaze detection processing 2 that does not require calibration.

### <11. Gaze Detection Device according to Third Embodiment of Present Technique>

Fig. 27A illustrates an eyeball irradiated with patterned light from a gaze detection device according to a third embodiment of the present technique. Fig. 27B illustrates an EVS image of the eyeball. Fig. 28 is a block diagram illustrating a function example of a gaze detection device 30 according to the third embodiment of the present technique.

The gaze detection device 30 according to the third embodiment has the same configuration as the gaze detection device 10 according to the first embodiment except that a reference pattern BP has multiple (e.g., three) patterns, each including at least two (e.g., two) lines crossing each other.

In the gaze detection device 30, the reference pattern BP includes at least two (e.g., three) patterns that are identical in shape and are oriented in different directions. In this case, patterned light corresponding to the reference pattern BP completely agrees with the reference pattern BP. Specifically, the patterned light corresponding to the reference pattern BP includes, for example, multiple (e.g., three) first to third pattern portions PL4A, PL4B, and PL4C that are identical in shape and are oriented in different direction as illustrated in Fig. 27A. In this case, the patterns of the reference pattern BP and the pattern portions of the patterned light are nearly V-shaped. Specifically, for example, two straight lines constituting the V shape of each pattern portion are not parallel to any one of two straight lines constituting the V shape of another pattern portion. The third pattern portion PL4C has one side extending nearly horizontally.

As illustrated in Fig. 27B, for example, a corneal reflection image PL4Ai of the first pattern portion PL4A, a corneal reflection image PL4Bi of the second pattern portion PL4B, and a corneal reflection image PL4Ci of the third pattern portion PL4C appear in an EVS image when an eyeball EB moves horizontally. The corneal reflection images PL4Ai and PL4Bi are each V-shaped with one intersection point. The corneal reflection images PL4Ci is a straight line.

In the gaze detection device 30, a patterned-light illumination system 100 includes first to third light sources 100a1, 100a2, and 100a3 as illustrated in Fig. 28. The first light source 100a1 is used for generating the first pattern portion PL4A. The second light source 100a2 is used for generating the second pattern portion PL4B. The third light source 100a3 is used for generating the third pattern portion PL4C. The first to third light sources 100a1, 100a2, and 100a3 are driven by a light source drive unit 100b.

Also in the gaze detection device 30, gaze detection processing 2 is performed. At this point, a feature point (an intersection point of the first pattern portion PL4A) of the corneal reflection image PL4Ai, a feature point (an intersection point of the second pattern portion PL4B) of the corneal reflection image PL4Bi, and a feature point (an intersection point of the third pattern portion PL4C) of the corneal reflection image PL4Ci are extracted, and the corneal center is calculated using the feature points.

According to the gaze detection device 30, at least two feature points of the corneal reflection image of the patterned light can be detected all the time, so that a gaze can be detected by the gaze detection processing 2 that does not require calibration.

### (Example of Method for Detecting Corneal-Reflection-Image Detection Unit)

In the foregoing embodiments and modification examples, a corneal-reflection-image detection unit 300a may extract, as a feature point, a pixel having increased in brightness in an EVS image. Fig. 29 is a block diagram illustrating function example 3 of the corneal-reflection-image detection unit. As illustrated in Fig. 29, function example 3 of the corneal-reflection-image detection unit 300a includes a brightness comparison unit 300a4 in addition to a feature point extraction unit 300a1 and an associating unit 300a2. The brightness comparison unit 300a4 compares the brightness of pixels in the current frame and the brightness of pixels in the preceding frame, the pixels constituting a feature point that is extracted by the feature point extraction unit 300a1 and is associated by the associating unit 300a2. The brightness comparison unit 300a4 then extracts only the pixel having increased in brightness from the pixels, as a pixel constituting the feature point. Thus, the feature point can be more strictly extracted.

### <12. Gaze Detection Device according to Fourth Embodiment of Present Technique>

Fig. 30 is a block diagram illustrating a function example of a gaze detection device 40 according to the fourth embodiment of the present technique.

As illustrated in Fig. 30, the gaze detection device 40 according to the fourth embodiment has nearly the same configuration as the gaze detection device 10 according to the first embodiment except that a gaze detection system 300 further includes a movement information estimation unit 300d.

The movement information estimation unit 300d estimates at least one of the amount of movement, the direction of movement, and the velocity of movement of an eyeball EB on the basis of the detection result in a corneal-reflection-image detection unit 300a.

### (Function Example 1 of Movement Information Estimation Unit)

Fig. 31 is a block diagram showing function example 1 of the movement information estimation unit 300d. The movement information estimation unit 300d in Fig. 31 includes a movement amount estimation unit 300d1 and a movement direction estimation unit 300d2. The movement amount estimation unit 300d1 can extract, for example, a feature point having decreased in brightness and a feature point having increased in brightness and estimate the amount of movement of the eyeball EB from the positional relationship between the two feature points. The movement direction estimation unit 300d2 can estimate the direction of movement of the eyeball EB from the positional relationship or estimate the direction of movement of the eyeball EB from, for example, the direction of an acute angle formed by two straight lines crossing at the feature point (intersection point) of a corneal reflection image PL4i or an event polarity (positive or negative) as shown in Fig. 32.

In the gaze detection device 40, a gaze detection unit 300c performs gaze detection processing 1. At this point, in a calibration step and/or a gaze detection step, the amount of movement and the direction of movement of the eyeball EB are associated with gaze in addition to the feature point of a corneal reflection image and the center of a pupil, the amount and direction of movement being estimated by the movement information estimation unit 300d, so that a gaze can be determined with higher accuracy.

### (Function Example 2 of Movement Information Estimation Unit)

Fig. 33 is a block diagram showing function example 2 of the movement information estimation unit 300d. The movement information estimation unit 300d in Fig. 33 includes the movement direction estimation unit 300d2 and a movement velocity estimation unit 300d3. The movement direction estimation unit 300d2 can extract, for example, a feature point having decreased in brightness and a feature point having increased in brightness and estimate the direction of movement of the eyeball EB from the positional relationship between the two feature points or estimate the direction of movement of the eyeball EB from, for example, the direction of an acute angle formed by two straight lines crossing at the feature point (intersection point) of the corneal reflection image PL4i or an event polarity (positive or negative) as shown in Fig. 32. The movement velocity estimation unit 300d3 may extract, for example, a feature point having decreased in brightness and a feature point having increased in brightness and estimate the velocity of movement of the eyeball EB from the positional relationship between the two feature points and the frame rate of an EVS.

In the function example 2 of the movement information estimation unit 300d in Fig. 33, saccade estimation is performed to estimate the target position (arrival position) of the eyeball EB from the direction of movement and the velocity of movement of the eyeball EB. Figs. 34A and 34B are explanatory drawings showing the principle of the function example 2 of the movement information estimation unit 300d. Figs. 34A and 34B show a change of an eyeball position and a change of an eyeball velocity over time with respect to the same movement of the eyeball. Figs. 34A and 34B show that a change of an eyeball position and a change of an eyeball velocity over time are associated with each other and the arrival time of the eyeball can be detected by detecting the maximum velocity.

### (Function Example 3 of Movement Information Estimation Unit)

Fig. 35 is a block diagram showing function example 3 of the movement information estimation unit 300d. The movement information estimation unit 300d in Fig. 35 estimates the position of a feature point that corresponds to an intersection point and has been undetected by the corneal-reflection-image detection unit 300a (has not appeared in an EVS image), from a feature point that corresponds to an intersection point and has been detected by the corneal-reflection-image detection unit 300a (has appeared in an EVS image), and then extracts the direction of movement of the eyeball EB from the estimation result. In this case, the movement information estimation unit 300d includes an undetected-intersection-point estimation unit 300d4 and a movement direction extraction unit 300d5. For example, as illustrated in Fig. 36, the undetected-intersection -point estimation unit 300d4 estimates the position of a feature point (e.g., a feature point of a corneal reflection image PL4Ai) that corresponds to an intersection point of the reference pattern BP and has been undetected by the corneal-reflection-image detection unit 300a (has not appeared in an EVS image), from the position of a feature point (e.g., a feature point of a corneal reflection image PL4Bi) that corresponds to an intersection point of the reference pattern BP and has been detected by the corneal-reflection-image detection unit 300a (for example, the broken line portion of Fig. 36 can be estimated), and then the undetected-intersection-point estimation unit 300d4 outputs the estimation result. This can increase the number of feature points, improve the accuracy of detection for the gaze detection processing 1, and also support the gaze detection processing 2.

### (Function Example of Pupil Detection Unit)

Fig. 37 is a block diagram showing a function example of a pupil detection unit 300b. The pupil detection unit 300b in Fig. 37 includes a movement information acquisition unit 300b1 and a pupil pattern estimation unit 300b2. The movement information acquisition unit 300b1 acquires movement information (e.g., at least one of the amount of movement, the direction of movement, and the velocity of movement) about the eyeball EB as the estimation result of the movement information estimation unit 300d. The pupil pattern estimation unit 300b2 estimates the pattern of a pupil from the movement information acquired about the eyeball EB by the movement information acquisition unit 300b1 and detects the pupil through pattern detection or the like.

According to the gaze detection device 40, the gaze detection system 300 includes the movement information estimation unit 300d, thereby further improving the accuracy of gaze detection.

### <13. Gaze Detection Devices according to Modification Examples 6 and 7 of Second Embodiment of Present Technique>

Figs. 38A and 38B illustrate eyeballs irradiated with patterned light from gaze detection devices according to modification examples 6 and 7 of the second embodiment of the present technique.

As illustrated in Fig. 38A, in the gaze detection device according to modification example 6, patterned light emitted to the eyeball EB includes rectangular first and second pattern portions PL6A and PL6B that are identical in shape (including a similar figure) and are oriented in different directions.

As illustrated in Fig. 38B, in the gaze detection device according to modification example 7, patterned light emitted to the eyeball EB includes a rectangular first pattern portion PL6 and a triangular second pattern portion PL1'.

### <14. Gaze Detection Devices according to Modification Examples 4 to 8 of First Embodiment of Present Technique>

Figs. 39A to 39E illustrate eyeballs irradiated with patterned light from gaze detection devices according to modification examples 4 to 8 of the first embodiment of the present technique. In the examples of Figs. 39A to 39E, all the reference patterns BP corresponding to patterned light extend radially (e.g., in a Y shape).

In modification example 4 in Fig. 39A, the patterned light PL2 completely agrees with the reference pattern BP.

In modification example 5 in Fig. 39B, the patterned light PL2 is shaped to extend along the reference pattern BP such that two of three straight lines cross each other with a straight line slightly separated (e.g., by ten pixels or less) from the intersection point of the two straight lines.

In modification example 6 in Fig. 39C, the patterned light PL2 is shaped to extend along the reference pattern BP such that a portion corresponding to the intersection point of the reference pattern BP is replaced with a figure (e.g., a circle).

In modification example 7 in Fig. 39D, the patterned light PL2 is shaped to extend along the reference pattern BP such that a portion corresponding to the intersection point of the reference pattern BP is removed.

In modification example 8 in Fig. 39E, the patterned light PL2 is shaped to extend along the reference pattern BP such that a portion corresponding to the intersection point of the reference pattern BP is removed and a figure (e.g., a circle) is placed in the removed portion.

### <15. Gaze Detection Device according to Modification Example 9 of First Embodiment of Present Technique>

Fig. 40 illustrates an eyeball irradiated with patterned light from a gaze detection device according to modification example 9 of the first embodiment of the present technique. In modification example 9 in Fig. 40, patterned light PL7 has three non-parallel straight lines crossing at a point. Four line segments extend radially from the intersection point of the three straight lines.

### <16. Gaze Detection Devices according to Modification Examples 10 to 12 of First Embodiment of Present Technique>

Figs. 41A to 41C illustrate eyeballs irradiated with patterned light from gaze detection devices according to modification examples 10 to 12 of the first embodiment of the present technique.

In modification example 10 in Fig. 41A, patterned light PL9 is A-shaped with three intersection points.

In modification example 11 in Fig. 41B, patterned light PL10 is A-shaped to have a laterally extending horizontal line with three intersection points.

In modification example 12 in Fig. 41C, patterned light PL11 is A-shaped to have a laterally extending horizontal line and two oblique lines extending upward with three intersection points.

### <17. Gaze Detection Devices according to Modification Examples 13 to 15 of First Embodiment of Present Technique>

Figs. 42A to 42C illustrate eyeballs irradiated with patterned light from gaze detection devices according to modification examples 13 to 15 of the first embodiment of the present technique.

In modification example 13 in Fig. 42A, patterned light PL12 is shaped such that a curve and two straight lines cross one another at a point and extend radially from the intersection point. The patterned light PL12 may be shaped such that a curve and two straight lines cross one another at multiple points.

In modification example 14 in Fig. 42B, patterned light PL13 is shaped such that two curves and a straight line cross one another at a point. The patterned light PL13 may be shaped such that two curves and a straight line cross one another at multiple points.

In modification example 15 in Fig. 42C, patterned light PL14 is shaped with three curves crossing at a point. The patterned light PL14 may be shaped with three curves crossing at multiple points.

### <18. Variation 1 of Patterned Light>

Fig. 43 shows variation 1 of patterned light suitable for the gaze detection device according to the present technique and corneal reflection images of the patterned light. Fig. 43 shows the patterned light shaped like stars in addition to polygons such as a triangle, a pentagon, and a hexagon. In Fig. 43, an EVS image in each direction (e.g., the horizontal direction, the vertical direction, and a diagonal direction) of movement of an eyeball is shown below each type of the patterned light. Fig. 43 shows that at least one feature point (intersection point) of a corneal reflection image can be detected from patterned light in any shape with respect to a horizontal movement, a vertical movement, and a diagonal movement of the eyeball.

### <19. Variation 2 of Patterned Light>

Fig. 44 shows variation 2 of patterned light suitable for the gaze detection device according to the present technique and corneal reflection images of the patterned light. Fig. 44 shows the patterned light shaped like star frames in addition to polygonal frames such as a triangular frame, a pentagonal frame, and a hexagonal frame. In Fig. 44, an EVS image in each direction (e.g., the horizontal direction, the vertical direction, and a diagonal direction) of movement of an eyeball is shown below each type of the patterned light. Fig. 44 shows that at least one feature point (intersection point) of a corneal reflection image can be detected from patterned light in any shape with respect to a horizontal movement, a vertical movement, and a diagonal movement of the eyeball.

### <20. Other Modification Examples of Present Technique>

For example, the number of light sources and the number of optical elements can be changed as appropriate. The number of light sources and the number of optical elements can be determined according to the number of divisions of patterned light (the number of pattern portions). Specifically, when light sources emit the pattern portions of patterned light, the number of light sources may be set equal to the number of pattern portions. When light emitted from light sources is shaped into pattern portions through optical elements, the number of optical elements may be set equal to the number of pattern portions and the number of light sources may be set equal to or smaller than the number of pattern portions.

For example, the imaging element 200a of the imaging system is not limited to an EVS and may be an ordinary image sensor.

In the gaze detection devices according to the foregoing embodiments and modification examples, the type, number, shape, and arrangement of the light sources and optical elements can be changed as appropriate.

For example, at least parts of the gaze detection devices according to the embodiments and modification examples may be combined without inconsistencies.

The present technique can also be configured as follows:
(1) A gaze detection device including: a patterned-light illumination system that irradiates an eyeball with patterned light;
   an imaging system that captures an image of the eyeball irradiated with the patterned light; and
   a gaze detection system that detects a gaze as the direction of the eyeball on the basis of the captured image of the imaging system, wherein
   the patterned light has a shape along a reference pattern having at least three lines not parallel to one another, the reference pattern having an intersection point where at least two lines of the at least three lines cross each other.
(2) The gaze detection device according to (1), wherein each of the at least three lines crosses at least one of the other lines.
(3) The gaze detection device according to (1) or (2), wherein each of the at least three lines crosses at least two of the other lines.
(4) The gaze detection device according to any one of (1) to (3), wherein the reference pattern has the at least three intersection points not located on the same straight line.
(5) The gaze detection device according to any one of (1) to (4), wherein the at least three lines cross one another at the same intersection point.
(6) The gaze detection device according to any one of (1) to (5), wherein the at least three lines form consecutive acute angles around the intersection point.
(7) The gaze detection device according to (5) or (6), wherein the at least three lines extend radially from the intersection point.
(8) The gaze detection device according to any one of (1) to (7), wherein the reference pattern has multiple patterns, each including the at least two lines crossing each other.
(9) The gaze detection device according to (8), wherein the multiple patterns include the at least two patterns that are identical in shape.
(10) The gaze detection device according to (9), wherein the multiple patterns are oriented in the same direction.
(11) The gaze detection device according to (9), wherein the multiple patterns are oriented of different directions.
(12) The gaze detection device according to (8), wherein the multiple patterns include the at least two patterns in different shapes.
(13) The gaze detection device according to any one of (8) to (12), wherein the patterned-light illumination system irradiates the eyeball with portion of the patterned light, the portion corresponding to the pattern where the intersection point is detectable among the multiple patterns.
(14) The gaze detection device according to any one of (1) to (13), wherein the gaze detection system includes a corneal-reflection-image detection unit that detects from the captured image a feature point of a corneal reflection image of the patterned light.
(15) The gaze detection device according to (14), wherein the corneal-reflection-image detection unit associates the intersection point and the feature point on the basis of information indicating an angle and/or a direction near the feature point of the corneal reflection image in the captured image.
(16) The gaze detection device according to (14) or (15), wherein the corneal-reflection-image detection unit extracts a pixel that has been increased in brightness in the captured image, as a pixel constituting the feature point.
(17) The gaze detection device according to any one of (14) to (16), wherein the gaze detection system includes a movement information estimation unit that estimates at least one of the amount of movement, the direction of movement, and the velocity of movement of the eyeball on the basis of a detection result in the corneal-reflection-image detection unit.
(18) The gaze detection device according to (17), wherein the movement information estimation unit estimates the position of the feature point that corresponds to the intersection point and that has not been detected by the corneal-reflection-image detection unit, from the feature point that corresponds to the intersection point and that has been detected by the corneal-reflection-image detection unit, and then extracts from the estimation result the direction of movement of the eyeball.
(19) The gaze detection device according to any one of (14) to (18), wherein the gaze detection system detects the gaze on the basis of at least a captured image of the imaging system and the detection result in the corneal-reflection-image detection unit.
(20) The gaze detection device according to any one of (1) to (19), wherein the patterned-light illumination system includes a light source that emits the patterned light.
(21) The gaze detection device according to any one of (1) to (19), wherein the patterned-light illumination system includes a light source and an optical element that shapes light from the light source to generate the patterned light.
(22) The gaze detection device according to any one of (1) to (21), wherein the imaging system includes an event sensor.

### [Reference Signs List]

10, 20, 30, 40 Gaze detection device
100 Patterned-light illumination system
100a Light source
100a1 First light source (light source)
100a2 Second light source (light source)
100a3 Third light source (light source)
200 Imaging system
300 Gaze detection system
300a Corneal-reflection-image detection unit
300d Movement information estimation unit

## Claims

1. A gaze detection device comprising:
a patterned-light illumination system that irradiates an eyeball with patterned light;
an imaging system that captures an image of the eyeball irradiated with the patterned light; and
a gaze detection system that detects a gaze as a direction of the eyeball on a basis of the captured image of the imaging system, wherein
the patterned light has a shape along a reference pattern having at least three lines not parallel to one another, the reference pattern having an intersection point where at least two lines of the at least three lines cross each other.

2. The gaze detection device according to claim 1, wherein each of the at least three lines crosses at least one of the other lines.

3. The gaze detection device according to claim 1, wherein each of the at least three lines crosses at least two of the other lines.

4. The gaze detection device according to claim 1, wherein the reference pattern has the at least three intersection points not located on the same straight line.

5. The gaze detection device according to claim 1, wherein the at least three lines cross one another at the same intersection point.

6. The gaze detection device according to claim 5, wherein the at least three lines form consecutive acute angles around the intersection point.

7. The gaze detection device according to claim 5, wherein the at least three lines extend radially from the intersection point.

8. The gaze detection device according to claim 1, wherein the reference pattern has multiple patterns, each including the at least two lines crossing each other.

9. The gaze detection device according to claim 8, wherein the multiple patterns include the at least two patterns that are identical in shape.

10. The gaze detection device according to claim 8, wherein the multiple patterns include the at least two patterns of different shapes.

11. The gaze detection device according to claim 8, wherein the patterned-light illumination system irradiates the eyeball with portion of the patterned light, the portion corresponding to the pattern where the intersection point is detectable among the multiple patterns.

12. The gaze detection device according to claim 1, wherein the gaze detection system includes a corneal-reflection-image detection unit that detects from the captured image a feature point of a corneal reflection image of the patterned light.

13. The gaze detection device according to claim 12, wherein the corneal-reflection-image detection unit associates the intersection point and the feature point on a basis of information indicating an angle and/or a direction near the feature point of the corneal reflection image in the captured image.

14. The gaze detection device according to claim 12, wherein the corneal-reflection-image detection unit extracts a pixel that has been increased in brightness in the captured image, as a pixel constituting the feature point.

15. The gaze detection device according to claim 12, wherein the gaze detection system includes a movement information estimation unit that estimates at least one of an amount of movement, a direction of movement, and a velocity of movement of the eyeball on a basis of a detection result in the corneal-reflection-image detection unit.

16. The gaze detection device according to claim 15, wherein the movement information estimation unit estimates a position of the feature point that corresponds to the intersection point and that has not been detected by the corneal-reflection-image detection unit, from the feature point that corresponds to the intersection point and that has been detected by the corneal-reflection-image detection unit, and then extracts from the estimation result the direction of movement of the eyeball.

17. The gaze detection device according to claim 12, wherein the gaze detection system detects the gaze on a basis of at least a captured image of the imaging system and a detection result in the corneal-reflection-image detection unit.

18. The gaze detection device according to claim 1, wherein the patterned-light illumination system includes a light source that emits the patterned light.

19. The gaze detection device according to claim 1, wherein the patterned-light illumination system includes:
a light source; and
an optical element that shapes light from the light source to generate the patterned light.

20. The gaze detection device according to claim 1, wherein the imaging system includes an event sensor.
